(19) Europäisches Patentamt
European Patent Office
Office européen des brevets

(11) EP 2 083 009 A1

(12) **EUROPÄISCHE PATENTANMELDUNG**

(43) Veröffentlichungstag:
**29.07.2009 Patentblatt 2009/31**

(51) Int Cl.:
***C07D 471/04*** ^(2006.01) ***A61K 31/437*** ^(2006.01)
***A61P 25/00*** ^(2006.01)

(21) Anmeldenummer: **08001093.7**

(22) Anmeldetag: **22.01.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Grünenthal GmbH**
**52078 Aachen (DE)**

(72) Erfinder:
- **Merla, Beatrix, Dr.**
  **52078 Aachen (DE)**
- **Oberbörsch, Stefan, Dr.**
  **52074 Aachen (DE)**
- **Kühnert, Sven, Dr.**
  **52355 Düren (DE)**
- **Bahrenberg, Gregor, Dr.**
  **52078 Aachen (DE)**
- **Kless, Achim, Dr.**
  **52074 Aachen (DE)**

(54) **Substituierte Tethrahydroimidazopyridin-Verbindungen und deren Verwendung in Arzneimitteln**

(57) Die vorliegende Erfindung betrifft substituierte Tetrahydroimidazopyridin-Verbindungen, Verfahren zu ihrer Herstellung, Arzneimittel enthaltend diese Verbindungen sowie die Verwendung dieser Verbindungen zur Herstellung von Arzneimitteln.

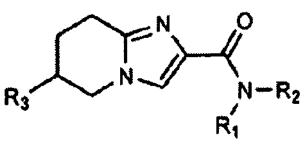

I

EP 2 083 009 A1

## Beschreibung

Die vorliegende Erfindung betrifft substituierte T etrahydroiniidazopyridin-Verbindungen, Verfahren zu ihrer Herstellung, Arzneimittel enthaltend diese Verbindungen sowie die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein pathophysiologisches Merkmal von chronischen Schmerzen besteht in der Überregbarkeit von Neuronen. Die neuronale Erregbarkeit wird entscheidend von der Aktivität von $K^+$ Kanälen beeinflusst, da diese das Ruhemembranpotential der Zelle und somit die Erregbarkeitsschwelle maßgeblich bestimmen. Heteromere $K^+$ Kanäle vom molekularen Subtyp KCNQ2/3 (Kv7.2/7.3) sind in Neuronen verschiedener Regionen des zentralen (Hippocampus, Amygdala) und peripheren (Hinterwurzelganglien) Nervensystems exprimiert und regulieren deren Erregbarkeit. Die Aktivierung von KCNQ2/3 $K^+$ Kanälen führt zu einer Hyperpolarisation der Zellmembran und damit einhergehend zu einer Abnahme der elektrischen Erregbarkeit dieser Neurone. KCNQ2/3 exprimierende Neurone der Hinterwurzelganglien sind an der Übertragung nociceptiver Erregungen von der Peripherie ins Rückenmark beteiligt (Passmore et al., J Neurosci. 2003; 23(18):7227-36). Dementsprechend konnte für den KCNQ2/3 Agonisten Retigabine eine analgetische Wirksamkeit in präklinischen Neuropathie- und Entzündungsschmerzmodellen nachgewiesen werden (Blackburn-Munro and Jensen, Eur J Pharmacol. 2003; 460(2-3):109-16; Dost et al., Naunyn Schmiedebergs Arch Pharmacol 2004; 369 (4): 382-390). Der KCNQ2/3 $K^+$ Kanal stellt somit einen geeigneten Ansatzpunkt zur Behandlung von Schmerz; insbesondere von Schmerz ausgewählt aus der Gruppe bestehend aus chronischem Schmerz, neuropathischem Schmerz, inflammatorischem Schmerz und muskulärem Schmerz (Nielsen et al., Eur J Pharmacol. 2004; 487(1-3): 93-103), insbesondere von neuropathischem und inflammatorischem Schmerz dar.

Darüber hinaus ist der KCNQ2/3 $K^+$ Kanal ein geeignetes Target für die Therapie einer Vielzahl weiterer Erkrankungen wie beispielsweise Migräne (US2002/0128277), kognitive Erkrankungen (Gribkoff, Expert Opin Ther Targets 2003; 7 (6): 737-748), Angstzuständen (Korsgaard et al., J Pharmacol Exp Ther. 2005, 14(1): 282-92), Epilepsie (Wickenden et al., Expert Opin Ther Pat 2004; 14(4): 457-469) und Harninkontinenz (Streng et al., J Urol 2004;172: 2054-2058).

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch KCNQ2/3 $K^+$ Kanäle vermittelt werden.

Überraschenderweise wurde nun gefunden, dass substituierte Tetrahydroimidazopyridin-Verbindungen der nachstehend angegebenen allgemeinen Formel I sich zur Behandlung von Schmerzen eignen und auch eine ausgezeichnete Affinität zum KCNQ2/3 $K^+$ Kanal aufweisen und sich daher zur Behandlung von Störungen oder Krankheiten eignen, die zumindest teilweise durch KCNQ2/3 $K^+$ Kanäle vermittelt werden.

In WO 2006/015737 wird eine Vielzahl von Verbindungen offenbart, unter denen sich auch substituierte Tetrahydroimidazopyridine befinden. Bei diesen Verbindungen handelt es sich um Dopaminrezeptorliganden, wobei jedoch bei diesen Verbindungen der Amid-Stickstoff immer über eine Alkyl- oder Cycloalkyl-Brücke mit einem phenylsubstituierten Piperazin verknüpft ist.

Ein Gegenstand der vorliegenden Erfindung sind daher substituierte Tetrahydroimidazopyridin-Verbindungen der allgemeinen Formel I,

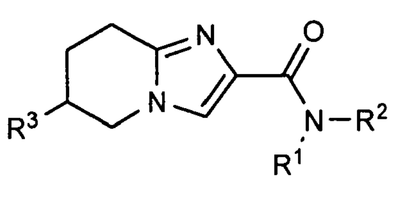

**I**

worin

$R^1$ und $R^2$ unabhängig voneinander für H; $C_{1-10}$-Alkyl, $C_{1-4}$-Alkyl-Aryl, $C_{1-4}$-Alkyl-Heteroaryl, $C_{1-4}$-Alkyl-$C_{3-8}$-Cycloalkyl, $C_{1-4}$-Alkyl-Heterocyclyl oder $C_{3-8}$-Cycloalkyl stehen,

wobei die Alkyl-, Aryl-, Heteroaryl-, Heterocyclyl- und Cycloalkylreste jeweils unsubstituiert oder einfach oder mehrfach substituiert sind und $R^1$ und $R^2$ nicht gleichzeitig H bedeuten;

oder die Reste $R^1$ und $R^2$ unter Einschluss des Stickstoffs einen vier bis achtgliedrigen, gegebenenfalls über eine $C_1$- oder $C_2$-Alkylkette verbrückten Heterocyclyl-Ring bilden, der ein weiteres Heteroatom enthalten und substituiert oder unsubstituiert sein kann,

und

$R^3$ Phenyl, einfach oder mehrfach substituiert oder unsubstituiert bedeutet,

mit der Maßgabe, dass wenn $R^1$ oder $R^2$ H, Alkyl oder Phenylalkyl bedeutet, der andere Rest $R^1$ oder $R^2$ nicht für eine Gruppe der Formel X1 steht

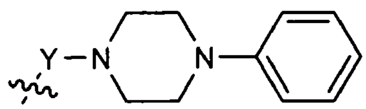

X1,

wobei der Phenylrest in der Formel X1 unsubstituiert oder substituiert sein kann und Y für eine $C_{2-5}$-Alkylkette oder $-(CH_2)_o-Z-(CH_2)_p-$ steht, wobei Z für Cyclopentyl, Cyclohexyl oder Cycloheptyl steht und o und p 0, 1, 2 oder 3 ist, wobei die Summe aus o und p höchstens 3 beträgt,

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die Ausdrücke "$C_{1-4}$-Alkyl", und "$C_{1-10}$-Alkyl"umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 4 C-Atomen bzw. 1 bis 10 C-Atome, d.h. $C_{1-4}$-Alkanyle, $C_{2-4}$-Alkenyle und $C_{2-4}$-Alkinyle bzw. $C_{1-10}$-Alkanyle, $C_{2-10}$-Alkenyle und $C_{2-10}$-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, Hexyl, Ethylenyl (Vinyl), Ethinyl, Propenyl ($-CH_2CH=CH_2$, $-CH=CH-CH_3$, $-C(=CH_2)-CH_3$), Propinyl ($-CH-C=CH$, $-C\equiv C-CH_3$), Butenyl, Butinyl, Pentenyl, Pentinyl, Hexenyl und Hexinyl umfasst. Besonders vorteihaft sind Methyl, Ethyl, n-Propyl und n-Butyl.

Der Ausdruck "Cycloalkyl" oder "$C_{3-8}$-Cycloalkyl" bedeutet für die Zwecke dieser Erfindung cyclische Kohlenwasserstoffe mit 3, 4, 5, 6, 7 oder 8 Kohlenstoffatomen, die gegebenenfalls auch verbrückt sein können, wobei die Kohlenwasserstoffe gesättigt oder ungesättigt (aber nicht aromatisch), unsubstituiert oder ein- oder mehrfach substituiert sein können. Vorteilhaft ist $C_{3-8}$-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält.

Der Begriff "Heterocyclyl" umfasst gesättigte oder ungesättigte (aber nicht aromatische) Cycloalkyle mit drei bis zehn, vorzugsweise vier bis acht Ringgliedern, die gegebenenfalls auch verbrückt sein können, in denen ein oder zwei Kohlenstoffatome durch ein Heteroatom S, N oder O ersetzt sind. Vorteilhaft sind Heterocyclyl-Reste aus der Gruppe Tetrahydropyranyl, Azabicyclo[3.2.1]octan, Dioxanyl, Dioxolanyl, Morpholinyl, Piperidinyl, Pyrazolinonyl und Pyrrolidinyl.

Der Ausdruck "Aryl" bedeutet im Sinne dieser Erfindung aromatische Kohlenwasserstoffe mit bis zu 14 Ringgliedern, u.a. Phenyle und Naphthyle. Die ArylReste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems mit bis zu 14 Ringgliedern sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Phtalazinyl, Pyrazolyl, Imidazolyl, Thiazolyl, Oxadiazolyl, Isoxazoyl, Oxazolyl, Triazolyl, Pyridyl, Pyridazinyl, Pyrimidinyl, Pyrazinyl, Indazolyl, Purinyl, Indolizinyl, Chinolinyl, Isochinolinyl, Chinazolinyl, Carbazolyl, Phenazinyl enthält, wobei die Bindung an die Verbindungen der allgemeinen Struktur I über jedes beliebige und mögliche Ringglied des Heteroaryl-Restes erfolgen kann. Besonders bevorzugt sind Pyridyl, Furyl und Thienyl.

Die Ausdrücke "über $C_{1-4}$-Alkyl gebundenes Aryl, Heteroaryl, Heterocyclyl oder Cycloalkyl" bedeuten für die Zwecke der vorliegenden Erfindung, daß $C_{1-4}$-Alkyl und Aryl bzw. Heteroaryl bzw. Heterocyclyl bzw. Cycloalkyl die oben definierten Bedeutungen haben und der Aryl- bzw. Heteroaryl- bzw. Heterocyclyl- bzw.

Cycloalkyl-Rest über eine $C_{1-4}$-Alkyl-Gruppe an die Verbindung der allgemeinen Struktur I gebunden ist. Die Alkylkette kann in allen Fällen gesättigt oder ungesättigt, verzweigt oder unverzweigt, unsubstituiert oder einfach oder mehrfach substituiert sein. Es ist vorteilhaft, wenn die Alkylkette unsubstituiert oder mit einer Methylgruppe substituiert ist. Besonders vorteilhaft im Sinne dieser Erfindung ist Phenyl, Benzyl und Phenethyl.

Im Zusammenhang mit "Alkyl", "Heterocyclyl" und "Cycloalkyl" versteht man unter dem Begriff "substituiert" im Sinne dieser Erfindung die Substitution eines Wasserstoffrestes durch F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, C(=O)O$C_{1-6}$-Alkyl, Phenyl oder Benzyl, wobei ein Substituent ggf. seinerseits substituiert sein kann, jedoch nicht mit einem weiteren Aryl- oder Heteroarylring. Unter mehrfach substituierten Resten sind solche Reste zu verstehen, die entweder an verschiedenen oder an gleichen Atomen mehrfach, z.B. zwei- oder dreifach, substituiert sind, beispielsweise dreifach am gleichen C-Atom wie im Falle von $CF_3$ oder -$CH_2CF_3$ oder an verschiedenen Stellen wie im Falle von -CH(OH)-CH=CH-$CHCl_2$. Die Mehrfachsubstitution kann mit dem gleichen oder mit verschiedenen Substituenten erfolgen.

In Bezug auf "Aryl", "Phenyl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH- $C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, C(=O)NH$C_{1-6}$-Alkyl; C(=O)-Aryl; C(=O)-N-Morpholin; C(=O)-Piperidin; (C=O)-Pyrrolidin; (C=O)-Piperazin; $NHSO_2C_{1-6}$-Alkyl, NHCO$C_{1-6}$-Alkyl, $CO_2H$, $CH_2SO_2$-Phenyl, $CO_2$-$C_{1-6}$-Alkyl, $OCF_3$, $SCF_3$, $CF_3$,

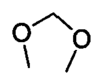 , 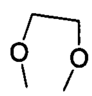 ,

$C_{1-6}$-Alkyl, Pyrolidinyl, Piperidinyl, Morpholinyl, Benzyloxy, Phenoxy, Phenyl, Pyridyl, Alkylaryl, Thienyl oder Furyl; an einem oder ggf. verschiedenen Atomen, wobei ein Substituent ggf. seinerseits substituiert sein kann, jedoch nicht mit einem weiteren Aryl- oder Heteroarylring. Die Mehrfachsubstitution erfolgt dabei mit dem gleichen oder mit unterschiedlichen Substituenten. Für "Aryl" oder "Heteroaryl" sind bevorzugte Substituenten F, Cl, $OCH_3$, $CF_3$, $OCF_3$, $SCF_3$ und $CH_3$.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Hippursäure, Phosphorsäure und/oder Asparaginsäure. Besonders bevorzugt sind die Zitronensäure und die Salzsäure.

Bevorzugt im Sinne dieser Erfindung sind substituierte Tetrahydroimidazopyridin-Verbindungen der allgemeinen Formel I,

worin

"Alkyl substituiert", "Heterocyclyl substituiert" und "Cycloalkyl substituiert" für die Substitution eines Wasserstoffrestes durch F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, C(=O)O$C_{1-6}$-Alkyl, Phenyl oder Benzyl steht,

und "Aryl substituiert", "Phenyl substituiert" und "Heteroaryl substituiert" für die ein-oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, 0-$C_{1-6}$Alkyl-OH, C(=O)-Aryl; C(=O)$C_{1-6}$-Alkyl, C(=O)NH$C_{1-6}$-Alkyl; C(=O)-N-Morpholin; C(=O)-Piperidin; (C=O)-Pyrrolidin; (C=O)-Piperazin; $NHSO_2C_{1-6}$-Alkyl, NHCO$C_{1-6}$-Alkyl, $CO_2H$, $CH_2SO_2$-Phenyl, $CO_2$-$C_{1-6}$-Alkyl, $OCF_3$, $CF_3$,

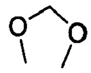,
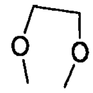,

$C_{1-6}$-Alkyl, Pyrolidinyl, Piperidinyl, Morpholinyl, Benzyloxy, Phenoxy, Phenyl, Pyridyl, Alkylaryl, Thienyl oder Furyl; steht.

Die Reste $C_{1-6}$-Alkyl, Pyrolidinyl, Piperidinyl, Morpholinyl, Benzyloxy, Phenoxy, Phenyl, Pyridyl, Alkylaryl, Thienyl oder Furyl können ihrerseits mit F, Cl, Methoxy, Ethoxy, $CF_3$, CN, $CH_3$, OH, $OCF_3$, $SCF_3$, oder $NO_2$ substituiert sein.

Weiterhin bevorzugt im Sinne dieser Erfindung sind substituierte Tetrahydroimidazopyridin-Verbindungen der allgemeinen Formel I, worin
$R^1$ und $R^2$ unabhängig voneinander für $C_{1-10}$-Alkyl, $C_{1-4}$-Alkyl-Aryl, $C_{1-4}$-Alkyl-Heteroaryl, $C_{1-4}$-Alkyl-$C_{3-8}$-Cycloalkyl, oder $C_{3-8}$-Cycloalkyl stehen,
wobei die Alkyl-, Aryl-, Heteroaryl- und Cycloalkylreste jeweils unsubstituiert oder einfach oder mehrfach substituiert sind.

Bevorzugt sind auch substituierte Tetrahydroimidazopyridin-Verbindungen der allgemeinen Formel I, worin die Reste $R^1$ und $R^2$ unter Einschluss des Stickstoffs einen vier bis achtgliedrigen, gegebenenfalls über eine $C_1$- oder $C_2$-Alkylkette verbrückten Ring bilden, der ein weiteres Heteroatom aus der Gruppe O, N oder S enthalten und substituiert oder unsubstituiert sein kann.

Besonders bevorzugt sind substituierte Tetrahydroimidazopyridin-Verbindungen der allgemeinen Formel I, worin
$R^1$ und $R^2$ unabhängig voneinander für H; Benzyl, Phenethyl, Methylpyridyl, Cyclopropyl, n-Pentyl, n-Butyl, n-Hexyl, sec-Butyl, Propylethyl oder Methylcyclohexyl,
jeweils unsubstituiert oder einfach oder mehrfach substituiert mit Methoxy, F, $CH_3$,
$CF_3$ oder

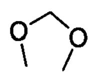,

stehen;
insbesondere

Benzyl, Phenethyl, Methylpyridyl, Cyclopropyl, 3,4-Dimethoxy-phenethyl, Benzo[1,3]dioxol-5-yl, 4-Fluorbenzyl, (1-Methyl)-propylphenyl, 3-Trifluormethylbenzyl, sec-Butyl, (1-Methyl)-benzyl, Methylcylohexyl oder (1-Methyl)-3,4-dimethylbenzyl, n-Butyl, n-Pentyl oder n-Hexyl.

Weiterhin besonders bevorzugt sind substituierte Tetrahydroimidazopyridin-Verbindungen der allgemeinen Formel I, worin

$R^1$ und $R^2$ einen fünf- bis siebengliedrigen Ring bilden, der ein weiteres Stickstoffatom enthalten kann und unsubsubstituiert oder einfach oder mehrfach substituiert mit $C(O)OC_2H_5$; $C(O)C_{1-6}$-Alkyl; Methyl, n-Butyl oder Acetyl; Phenyl oder Benzyl jeweils unsubstituiert oder substituiert mit Phenyl F, Cl, Br, I, CN, $CH_3$, $C_2H_5$, $NH_2$, $NO_2$, SH, $CF_3$, OH, $OCH_3$, $OCF_3$, $SCF_3$, $SCH_3$, $OC_2H_5$ oder $N(CH_3)_2$ ist,

Ganz besonders bevorzugt sind substituierte Tetrahydroimidazopyridin-Verbindungen der allgemeinen Formel I, worin die Gruppierung $NR^1R^2$ für

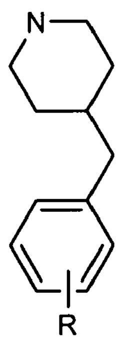

oder

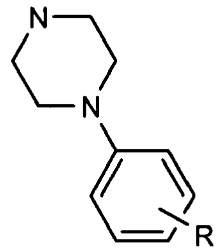

wobei R H, Phenyl F, Cl, Br, I, CN, $CH_3$, $C_2H_5$, $NH_2$, $NO_2$, SH, $CF_3$, OH, $OCH_3$, $OCF_3$, $SCF_3$, $SCH_3$, $OC_2H_5$ oder $N(CH_3)_2$ bedeutet,
insbesondere

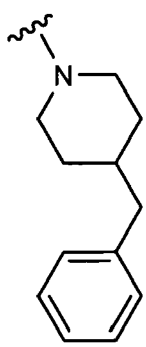

oder

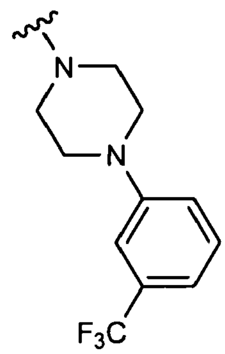

.

Weiterhin bevorzugt sind Tetrahydroimidazopyridin-Derivate, worin $R^3$ Phenyl, unsubstituiert oder einfach oder mehrfach substituiert mit F, Cl, Br, I, CN, $CH_3$, $C_2H_5$, $NH_2$, $NO_2$, SH, $CF_3$, OH, $OCH_3$, $OCF_3$, $SCF_3$, $SCH_3$, Phenoxy, $OC_2H_5$ oder $N(CH_3)_2$ bedeutet,
**[0030]** insbesondere Phenyl einfach oder mehrfach substituiert mit F, $OCH_3$, $CH_3$ oder Phenoxy.
Besonders bevozugt sind Tetrahydroimidazopyridin-Derivate, worin $R^3$ für

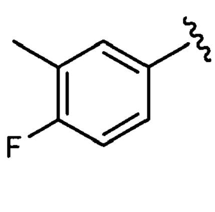

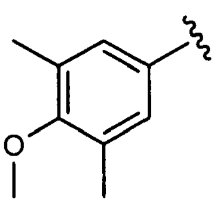

oder

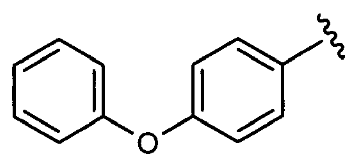

steht.
Am meisten bevorzugt sind Tetrahydroimidazopyridin-Derivate aus der Gruppe

1 (4-Benzylpiperidin-1-yl)(6-phenyl-5,6,7,8-tetrahydroimidazo[1,2-a]pyridin -2-yl)methanon
2 N-Benzyl-N-phenethyl-6-phenyl-5,6,7,8-tetrahydroimidazo[1,2-a]pyridin-2-carboxamid
3 (6-Phenyl-5,6,7,8-tetrahydroimidazo[1,2-a]pyridin-2-yl)(1,3,3-trimethyl-6-azabicyclo[3.2.1]octan-6-yl)methanon
4 (6-(3-Fluorphenyl)-5,6,7,8-tetrahydroimidazo[1,2-a]pyridin-2-yl)(1,3,3-trimethyl-6-azabicyclo[3.2.1]octan-6-yl) methanon
5 N-Benzyl-6-(4-methoxy-3,5-dimethylphenyl)-5,6,7,8-tetrahydroimidazo[1,2-a]pyridin-2-carboxamid

6 (4-(2-Fluorphenyl)piperazin-1-yl)(6-(4-methoxy-3,5-dimethylphenyl)-5,6,7,8-tetrahydroimidazo[1,2-a]pyridin-2-yl)methanon

7 (6-(5-Fluor-2-methoxyphenyl)-5,6,7,8-tetrahydroimidazo[1,2-a]pyridin-2-yl)(4-(3-(trifluormethyl)phenyl)piperazin-1-yl)methanon

8 (4-Benzylpiperidin-1-yl)(6-(5-fluor-2-methoxyphenyl)-5,6,7,8-tetrahydroimidazo[1,2-a]pyridin-2-yl)methanon

9 (6-(5-Fluor-2-methoxyphenyl)-5,6,7,8-tetrahydroimidazo[1,2-a]pyridin-2-yl)(1,3,3-trimethyl-6-azabicyclo[3.2.1]octan-6-yl)methanon

10 (6-(4-Methoxy-3,5-dimethylphenyl)-5,6,7,8-tetrahydroimidazo[1,2-a]pyridin-2-yl)(4-(2-methoxyphenyl)piperazin-1-yl)methanon

11 (4-Benzylpiperidin-1-yl)(6-(4-ethylphenyl)-5,6,7,8-tetrahydroimidazo[1,2-a]pyridin-2-yl)methanon

12 (6-(2,5-Dimethoxyphenyl)-5,6,7,8-tetrahydroimidazo[1,2-a]pyridin-2-yl)(1,3,3-trimethyl-6-azabicyclo[3.2.1]octan-6-yl)methanon

13 (6-(4-Fluor-3-methylphenyl)-5,6,7,8-tetrahydroimidazo[1,2-a]pyridin-2-yl)(4-(2-fluorphenyl)piperazin-1-yl)methanon

14 6-(4-Fluor-3-methylphenyl)-N-(1-phenylethyl)-5,6,7,8-tetrahydroimidazo[1,2-a]pyridin-2-carboxamid

15 6-(4-Methoxy-3,5-dimethylphenyl)-N-pentyl-5,6,7,8-tetrahydroimidazo[1,2-a]pyridin-2-carboxamid

16 N-Hexyl-6-(4-methoxy-3,5-dimethylphenyl)-5,6,7,8-tetrahydroimidazo[1,2-a]pyridin-2-carboxamid

17 6-(4-Methoxy-3,5-dimethylphenyl)-N-(3-(trifluormethyl)benzyl)-5,6,7,8-tetrahydroimidazo[1,2-a]pyridin-2-carboxamid

18 N-sec-Butyl-6-(4-phenoxyphenyl)-5,6,7,8-tetrahydroimidazo[1,2-a]pyridin-2-carboxamid

19 N-Butyl-6-(4-phenoxyphenyl)-5,6,7,8-tetrahydroimidazo[1,2-a]pyridin-2-carboxamid

20 Ethyl 1-(6-(4-phenoxyphenyl)-5,6,7,8-tetrahydroimidazo[1,2-a]pyridin-2-carbonyl)piperidin-4-carboxylat

21 (4-Methylpiperidin-1-yl)(6-(4-phenoxyphenyl)-5,6,7,8-tetrahydroimidazo[1,2-a]pyridin-2-yl)methanon

22 Ethyl 1-(6-(4-phenoxyphenyl)-5,6,7,8-tetrahydroimidazo[1,2-a]pyridin-2-carbonyl)piperidine-3-carboxylate

23 1-(4-(6-(4-Phenoxyphenyl)-5,6,7,8-tetrahydroimidazo[1,2-a]pyridin-2-carbonyl)piperazin-1-yl)ethanon

24 6-(4-Phenoxyphenyl)-N-(pyridin-3-ylmethyl)-5,6,7,8-tetrahydroimidazo[1,2-a]pyridin-2-carboxamid

25 (6-(4-Phenoxyphenyl)-5,6,7,8-tetrahydroimidazo[1,2-a]pyridin-2-yl)(pyrrolidin-1-yl)methanon

26 N-Cyclopropyl-6-(4-phenoxyphenyl)-5,6,7,8-tetrahydroimidazo[1,2-a]pyridin-2-carboxamid

27 N-(3,4-Dimethoxyphenethyl)-6-(4-phenoxyphenyl)-5,6,7,8-tetrahydroimidazo[1,2-a]pyridin-2-carboxamid

28 N-Pentyl-6-(4-phenoxyphenyl)-5,6,7,8-tetrahydroimidazo[1,2-a]pyridin-2-carboxamid

29 Pyrrolidin-1-yl(6-m-tolyl-5,6,7,8-tetrahydroimidazo[1,2-a]pyridin-2-yl)methanon

30 (6-(3,5-Bis(trifluormethyl)phenyl)-5,6,7,8-tetrahydroimidazo[1,2-a]pyridin-2-yl)(pyrrolidin-1-yl)methanon

31 Pyrrolidin-1-yl(6-(3-(trifluormethyl)phenyl)-5,6,7,8-tetrahydroimidazo[1,2-a]pyridin-2-yl)methanon

32 (4-(2-Fluorphenyl)piperazin-1-yl)(6-(3-(trifluormethyl)phenyl)-5,6,7,8-tetrahydroimidazo[1,2-a]pyridin-2-yl)methanon

33 (6-(2,4-Dimethoxyphenyl)-5,6,7,8-tetrahydroimidazo[1,2-a]pyridin-2-yl)(pyrrolidin-1-yl)methanon

34 Pyrrolidin-1-yl(6-o-tolyl-5,6,7,8-tetrahydroimidazo[1,2-a]pyridin-2-yl)methanon

35 (6-o-Tolyl-5,6,7,8-tetrahydroimidazo[1,2-a]pyridin-2-yl)(4-(3-(trifluormethyl)phenyl)piperazin-1-yl)methanon

36 (6-(2,5-Difluorphenyl)-5,6,7,8-tetrahydroimidazo[1,2-a]pyridin-2-yl)(pyrrolidin-1-yl)methanon

37 (6-(4-Ethylphenyl)-5,6,7,8-tetrahydroimidazo[1,2-a]pyridin-2-yl)(pyrrolidin-1-yl)methanon

38 (6-(4-Fluorphenyl)-5,6,7,8-tetrahydroimidazo[1,2-a]pyridin-2-yl)(1,3,3-trimethyl-6-azabicyclo[3.2.1]octan-6-yl)methanon

39 6-(4-Methoxyphenyl)-N-(3-(trifluormethyl)benzyl)-5,6,7,8-tetrahydroimidazo[1,2-a]pyridin-2-carboxamid

40 6-(4-Ethylphenyl)-N-phenEthyl-5,6,7,8-tetrahydroimidazo[1,2-a]pyridin-2-carboxamid

41 (4-Benzylpiperidin-1-yl)(6-(4-fluorphenyl)-5,6,7,8-tetrahydroimidazo[1,2-a]pyridin-2-yl)methanon

42 N-(Benzo[d][1,3]dioxol-5-ylmethyl)-6-(3,5-bis(trifluormethyl)phenyl)-5,6,7,8-tetrahydroimidazo[1,2-a]pyridin-2-carboxamid

43 N-Benzyl-6-(3,5-bis(trifluormethyl)phenyl)-5,6,7,8-tetrahydroimidazo[1,2-a]pyridin-2-carboxamid

44 (6-(4-Methoxy-3,5-dimethylphenyl)-5,6,7,8-tetrahydroimidazo[1,2-a]pyridin-2-yl)(4-(4-methoxyphenyl)piperazin-1-yl)methanon

45 (4-(2-Ethoxyphenyl)piperazin-1-yl)(6-m-tolyl-5,6,7,8-tetrahydroimidazo[1,2-a]pyridin-2-yl)methanon

46 (6-(2,4-Dimethoxyphenyl)-5,6,7,8-tetrahydroimidazo[1,2-a]pyridin-2-yl)(4-(3-(trifluormethyl)phenyl)piperazin-1-yl)methanon

47 (6-(4-Fluorphenyl)-5,6,7,8-tetrahydroimidazo[1,2-a]pyridin-2-yl)(4-(3-(trifluormethyl)phenyl)piperazin-1-yl)methanon

48 (6-(3-Fluorphenyl)-5,6,7,8-tetrahydroimidazo[1,2-a]pyridin-2-yl)(4-(3-(trifluormethyl)phenyl)piperazin-1-yl)methanon

49 N-(4-Fluorbenzyl)-6-(4-methoxy-3,5-dimethylphenyl)-5,6,7,8-tetrahydroimidazo[1,2-a]pyridin-2-carboxamid

50 (4-Benzylpiperidin-1-yl)(6-(3-methoxyphenyl)-5,6,7,8-tetrahydroimidazo[1,2-a]pyridin-2-yl)methanon
51 6-(3-Fluorphenyl)-N-(4-phenylbutan-2-yl)-5,6,7,8-tetrahydroimidazo[1,2-a]pyridin-2-carboxamid
52 6-(4-Ethylphenyl)-N-(4-phenylbutan-2-yl)-5,6,7,8-tetrahydroimidazo[1,2-a]pyridin-2-carboxamid
53 (6-Phenyl-5,6,7,8-tetrahydroimidazo[1,2-a]pyridin-2-yl)(4-(3-(trifluormethyl)phenyl)piperazin-1-yl)methanon
54 (6-(4-Methoxy-3,5-dimethylphenyl)-5,6,7,8-tetrahydroimidazo[1,2-a]pyridin-2-yl)(4-(3-(trifluormethyl)phenyl) piperazin-1-yl)methanon
55 (4-Benzylpiperidin-1-yl)(6-(4-methoxy-3,5-dimethylphenyl)-5,6,7,8-tetrahydroimidazo[1,2-a]pyridin-2-yl)methanon
56 (6-(4-Methoxy-3,5-dimethylphenyl)-5,6,7,8-tetrahydroimidazo[1,2-a]pyridin-2-yl)(1,3,3-trimethyl-6-azabicyclo [3.2.1]octan-6-yl)methanon
57 4-Benzylpiperidin-1-yl)(6-(4-fluor-3-methylphenyl)-5,6,7,8-tetrahydroimidazo[1,2-a]pyridin-2-yl)methanon
58 (6-(4-Fluor-3-methylphenyl)-5,6,7,8-tetrahydroimidazo[1,2-a]pyridin-2-yl)(4-(3-(triFluormethyl)phenyl)piperazin-1-yl)methanon
59 (4-Benzhydrylpiperazin-1-yl)(6-(4-methoxy-3,5-dimethylphenyl)-5,6,7,8-tetrahydroimidazo[1,2-a]pyridin-2-yl)methanon
60 6-(3-Methoxyphenyl)-N-(4-phenylbutan-2-yl)-5,6,7,8-tetrahydroimidazo[1,2-a]pyridin-2-carboxamid
61 (4-Benzylpiperidin-1-yl)(6-(3-fluorphenyl)-5,6,7,8-tetrahydroimidazo[1,2-a]pyridin-2-yl)methanon
62 (6-(4-Fluor-3-methylphenyl)-5,6,7,8-tetrahydroimidazo[1,2-a]pyridin-2-yl)(4-(4-(trifluormethyl)phenyl)piperazin-1-yl)methanon
63 (6-(4-Fluor-3-methylphenyl)-5,6,7,8-tetrahydroimidazo[1,2-a]pyridin-2-yl)(4-(4-fluorphenyl)piperazin-1-yl)methanon
64 (6-(4-Fluor-3-methylphenyl)-5,6,7,8-tetrahydroimidazo[1,2-a]pyridin-2-yl)(4-(4-methylphenyl)piperazin-1-yl)methanon
65 (6-(4-Fluor-3-methylphenyl)-5,6,7,8-tetrahydroimidazo[1,2-a]pyridin-2-yl)(4-(3-methylphenyl)piperazin-1-yl)methanon
66 (4-Butylpiperazin-1-yl)(6-(4-fluor-3-methylphenyl)-5,6,7,8-tetrahydroimidazo[1,2-a]pyridin-2-yl)methanon
67 (4-Benzhydrylpiperidin-1-yl)(6-(4-fluor-3-methylphenyl)-5,6,7,8-tetrahydroimidazo[1,2-a]pyridin-2-yl)methanon
68 N-(Cyclohexylmethyl)-6-(4-fluor-3-methylphenyl)-5,6,7,8-tetrahydroimidazo[1,2-a]pyridin-2-carboxamid
69 (6-(4-Methoxyphenyl)-5,6,7,8-tetrahydroimidazo[1,2-a]pyridin-2-yl)(4-(3-(trifluormethyl)phenyl)piperazin-1-yl) methanon
70 (4-Benzylpiperidin-1-yl)(6-m-tolyl-5,6,7,8-tetrahydroimidazo[1,2-a]pyridin-2-yl)methanon
71 (6-(3-Methoxyphenyl)-5,6,7,8-tetrahydroimidazo[1,2-a]pyridin-2-yl)(4-(3-(trifluormethyl)phenyl)piperazin-1-yl) methanon
72 6-(4-Fluor-3-methylphenyl)-N-(4-phenylbutan-2-yl)-5,6,7,8-tetrahydroimidazo[1,2-a]pyridin-2-carboxamid
73 4-Benzylpiperidin-1-yl)(6-p-tolyl-5,6,7,8-tetrahydroimidazo[1,2-a]pyridin-2-yl)methanon
74 (6-p-Tolyl-5,6,7,8-tetrahydroimidazo[1,2-a]pyridin-2-yl)(4-(3-(trifluormethyl)phenyl)piperazin-1-yl)methanon
75 (6-m-Tolyl-5,6,7,8-tetrahydroimidazo[1,2-a]pyridin-2-yl)(4-(3-(trifluormethyl)phenyl)piperazin-1-yl)methanon
76 (4-Benzylpiperidin-1-yl)(6-(4-methoxyphenyl)-5,6,7,8-tetrahydroimidazo[1,2-a]pyridin-2-yl)methanon
77 (6-(3,5-Dimethylphenyl)-5,6,7,8-tetrahydroimidazo[1,2-a]pyridin-2-yl)(4-(3-(trifluormethyl)phenyl)piperazin-1-yl) methanon
78 N-(1-(3,5-Dimethylphenyl)ethyl)-6-(4-fluor-3-methylphenyl)-5,6,7,8-tetrahydroimidazo[1,2-a]pyridin-2-carboxamid
79 6-(3,4-Difluorphenyl)-5,6,7,8-tetrahydroimidazo[1,2-a]pyridin-2-yl)(4-(3-(trifluormethyl)phenyl)piperazin-1-yl)methanon
80 (4-Benzylpiperazin-1-yl)(6-(4-fluor-3-methylphenyl)-5,6,7,8-tetrahydroimidazo[1,2-a]pyridin-2-yl)methanon
81 (6-(4-Phenoxyphenyl)-5,6,7,8-tetrahydroimidazo[1,2-a]pyridin-2-yl)(4-(3-(trifluormethyl)phenyl)piperazin-1-yl) methanon
82 6-(4-(Benzyloxy)phenyl)-5,6,7,8-tetrahydroimidazo[1,2-a]pyridin-2-yl)(4-(3-(trifluormethyl)phenyl)piperazin-1-yl) methanon

Die erfindungsgemäßen substituierten Tetrahydroimidazopyridin-Verbindungen sowie jeweils die entsprechenden Säuren, Basen, Salze und Solvate eignen sich als pharmazeutische Wirkstoffe in Arzneimitteln.

Ein weiterer Gegenstand der vorliegenden Erfindung ist daher ein Arzneimittel enthaltend wenigstens eine erfindungsgemäße substituierte Tetrahydroimidazopyridin-Verbindung der allgemeinen Formel I, worin die Reste $R^1$-$R^3$ die oben angegebene Bedeutung haben, sowie ggf. einen oder mehrere pharmazeutisch verträgliche Hilfsstoffe.

Diese erfindungsgemäßen Arzneimittel eignen sich zur Beeinflussung von KCNQ2/3-Kanälen und üben eine agonistische oder antagonistische, insbesondere eine agonistische Wirkung aus.

Bevorzugt eignen sich die erfindungsgemäßen Arzneimittel zur Behandlung von Störungen oder Krankheiten, die zumindest teilweise durch KCNQ2/3-Kanäle vermittelt werden.

Bevorzugt eignet sich das erfindungsgemäße Arzneimittel zur Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz, Migräne; Epilepsie, Angstzuständen und Harninkontinenz. Besonders bevorzugt eignen sich die erfindungsgemäßen Arzneimittel zur Behandlung von Schmerz, ganz besonders bevorzugt von chronischem Schmerz, neuropathischem Schmerz, inflammatorischem Schmerz und muskulärem Schmerz.

Weiterhin eignen sich die erfindungsgemäßen Verbindungen bevorzugt zur Behandlung von Epilepsie.

Ein weiterer Gegenstand der vorliegenden Erfindung ist die Verwendung wenigstens einer erfindungsgemäßen substituierten Tetrahydroimidazopyridin-Verbindung sowie ggf. eines oder mehrerer pharmazeutisch verträglicher Hilfsstoffe zur Herstellung eines Arzneimittels zur Behandlung von Störungen oder Krankheiten, die zumindest teilweise durch KCNQ2/3-Kanäle vermittelt werden.

Bevorzugt ist die Verwendung wenigstens einer erfindungsgemäßen substituierten Tetrahydroimidazopyridin-Verbindung sowie ggf. eines oder mehrerer pharmazeutisch verträglicher Hilfsstoffe zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Migräne; Epilepsie, Angstzuständen und Harninkontinenz.

Besonders bevorzugt ist die Verwendung wenigstens einer erfindungsgemäßen substituierten Tetrahydroimidazopyridin-Verbindung sowie ggf. eines oder mehrerer pharmazeutisch verträglicher Hilfsstoffe zur Herstellung eines Arzneimittels zur Behandlung von Schmerz, ganz besonders bevorzugt von chronischem Schmerz, neuropathischem Schmerz, inflammatorischem Schmerz und muskulärem Schmerz. Weiterhin besonders bevorzugt ist die Verwendung wenigstens einer erfindungsgemäßen substituierten Tetrahydroimidazopyridin-Verbindung sowie ggf. eines oder mehrerer pharmazeutisch verträglicher Hilfsstoffe zur Herstellung eines Arzneimittels zur Behandlung von Epilepsie.

Die Wirksamkeit gegen Schmerz kann beispielsweise in den unten beschriebenen Bennett- bzw. Chung-Modell gezeigt werden. Die Wirksamkeit gegen Epilepsie kann beispielsweise im DBA/2 Maus Modell (De Sarro et al., Naunyn-Schmiedeberg's Arch. Pharmacol. 2001, 363, 330-336) nachgewiesen werden.

Ein weiterer Gegenstand der vorliegenden Erfindung ist ein Verfahren zur Herstellung der erfindungsgemäßen substituierten Tetrahydroimidazopyridin-Verbindungen. Die in den vorstehend beschriebenen Umsetzungen zum Einsatz kommenden Chemikalien und Reaktionskomponenten sind käuflich erhältlich oder können jeweils nach dem Fachmann bekannten Methoden hergestellt werden.

X = Cl, Br, I

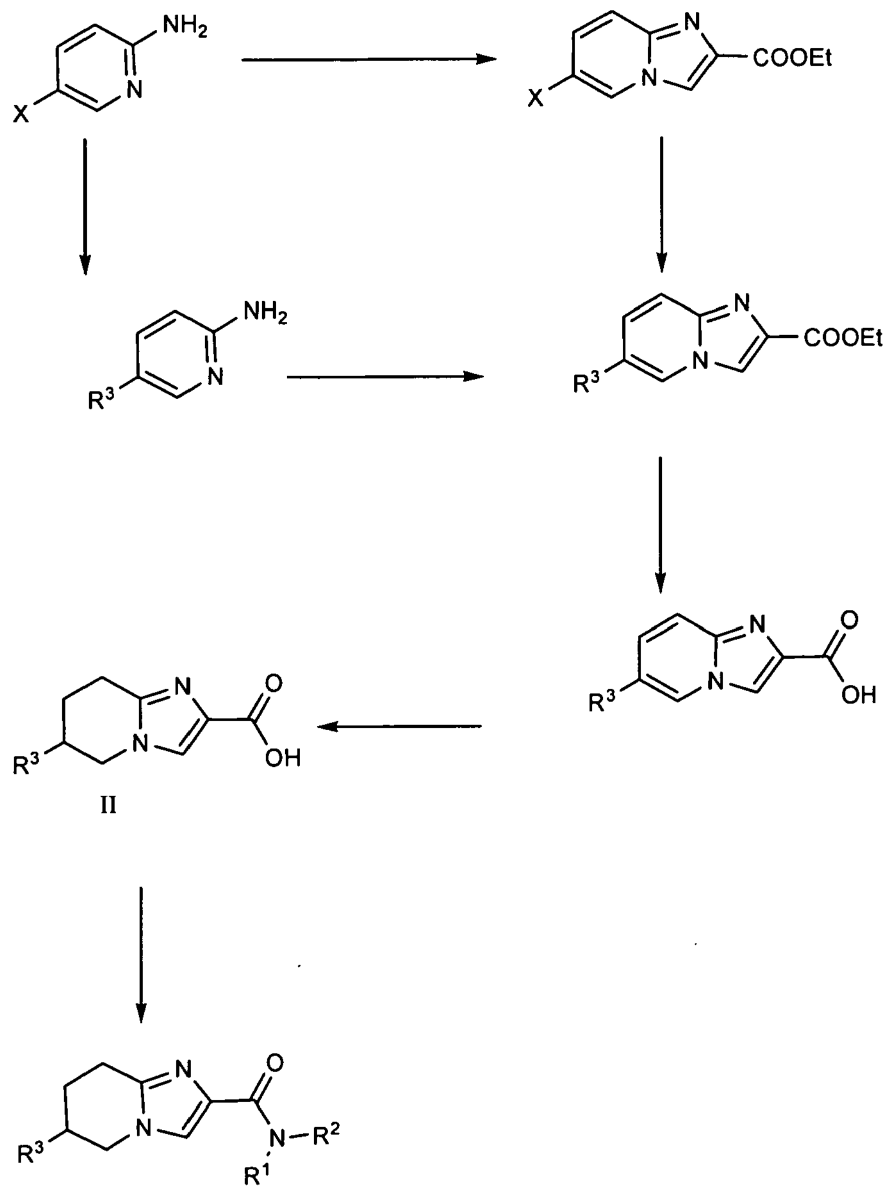

II

I

Ausgehend von einem entsprechend iod-, brom- oder chlorsubstituierten 2-Aminopyridin erfolgt der Ringschluss durch Umsetzung mit 3-Bromo-2-oxopropionsäureethylester in einem organischen Lösungsmittel, beispielsweise Ethanol, Methanol, THF, 1,2-Dimethoxyethan, Aceton, Wasser oder Chloroform bei Temperaturen zwischen 0˚C und 80˚C bei einer Reaktionszeit von 2-48 h. Alternativ kann die Ringschlussreaktion auch ausgehend von dem primär gebildeten 2-Aminopyridiniumbromid durch Erhitzen in Methanol erfolgen.

Die anschließende Suzuki-Reaktion erfolgt durch Umsetzung des entsprechenden iod-, brom- oder chlorsubstituierten Imidazols mit Phenylboronsäuren oder Phenylboronsäureestern in Lösungsmitteln, wie Methanol, Ethanol, 1-Propanol, Ethylenglycol, Wasser, 1,2-Dimethoxyethan, THF, Dioxan, Acetonitril, DMF, Benzol, Toluol, oder Xylol, die auch als Gemische vorliegen können, unter Verwendung eines Katalysators, einer Base und gegebenenfalls eines Additivs.

Als Katalysatoren können unter anderem $Pd(PPh_3)_4$, $Pd(dba)_2$, $PdCl_2(dppf)_2$, $Pd(P(o\text{-}Tolyl)_3)_4$, $PdCl_2(PPh_3)_2$, $PdCl_2(P(Cy)_3)_2$, $Pd(OAc)_2(P(o\text{-}Tolyl)_3)_2$ verwendet werden. Außerdem kann eine Mischung aus $Pd(OAc)_2$ / $PPh_3$, Pd auf Aktivkohle / $PPh_3$ oder $(NH_4)_2PdCl_4$ als Katalysator verwendet werden. Als weiterer Ligand eignet sich Tri-tert.-butylphosphin.

Als Basen können sowohl organische als auch anorganische Basen Verwendung finden. Als organische Basen eignen sich z.B. TEA oder Natrium-tert.-Butylat. Als anorganische Basen eignen sich beispielsweise Natrium-, Natriumhydrogen-, Kalium-, Cäsium- oder Silbercarbonat, Barium-, Natrium-, oder Kaliumhydroxid oder Kaliumphosphat.

Als Additive können beispielsweise EDTA, Tetrabutylammoniumbromid, Lithiumchlorid, Kaliumfluorid oder Methylcyclohexan verwendet werden.
Die Reaktionstemperatur liegt zwischen Raumtemperatur und 80˚C, bei einer Reaktionszeit von 1,5 -72 h.
Die Reaktion kann auch in der Mikrowelle durchgeführt werden.

Ein anderer Reaktionsweg geht von iod-, brom- oder chlorsubstituierten 2-Aminopyridinen aus, die zunächst in einer Suzuki-Reaktion zu den phenylsubstituierten Aminopyridinen umgesetzt werden.

Die Suzuki-Reaktion erfolgt durch Umsetzung des entsprechenden iod-, brom- oder chlorsubstituierten 2-Aminopyridins mit Boronsäuren oder Boronsäureestern in Lösungsmitteln, beispielsweise Methanol, Ethanol, 1-Propanol, Ethylenglycol, Wasser, 1,2-Dimethoxyethan, THF, Dioxan, Acetonitril, DMF, Benzol, Toluol, oder Xylol, die auch als Gemische vorliegen können, unter Verwendung eines Katalysators, einer Base und gegebenenfalls eines Additivs.
Als Katalysatoren können unter anderem $Pd(PPh_3)_4$, $Pd(dba)_2$, $PdCl_2(dppf)_2$, $Pd(P(o\text{-}Tolyl)_3)_4$, $PdCl_2(PPh_3)_2$, $PdCl_2(P(Cy)_3)_2$, $Pd(OAc)_2(P(o\text{-}Tolyl)_3)_2$ verwendet werden. Außerdem kann eine Mischung aus $Pd(OAc)_2$ / $PPh_3$, Pd auf Aktivkohle / $PPh_3$ oder $(NH_4)_2PdCl_4$ als Katalysator verwendet werden. Als weiterer Ligand eignet sich Tri-tert.-butylphosphin.
Als Basen können sowohl organische als auch anorganische Basen Verwendung finden. Als organische Basen eignen sich z.B. TEA oder Natrium-tert.-Butylat. Als anorganische Basen eignen sich beispielsweise Natrium-, Natriumhydrogen-, Kalium-, Cäsium- oder Silbercarbonat, Barium-, Natrium-, oder Kaliumhydroxid oder Kaliumphosphat.
Als Additive können beispielsweise EDTA, Tetrabutylammoniumbromid, Lithiumchlorid, Kaliumfluorid oder Methylcyclohexan verwendet werden.
Die Reaktionstemperatur liegt zwischen Raumtemperatur und 80˚C, bei einer Reaktionszeit von 1,5 -72 h.
Die Reaktion kann auch in der Mikrowelle durchgeführt werden.

Der Ringschluß kann durch Umsetzung des phenylsubstituierten Aminopyridins mit 3-Bromo-2-oxopropionsäureethylester in einem organischen Lösungsmittel, beispielsweise Ethanol, Methanol, THF, 1,2-Dimethoxyethan, Aceton, Wasser oder Chloroform bei Temperaturen zwischen 0˚C und 80˚C bei einer Reaktionszeit von 2-48 h. Alternativ kann die Ringschlussreaktion auch ausgehend von dem primär gebildeten 2-Aminopyridiniumbromid durch Erhitzen in Methanol erfolgen.

Die anschließende Esterspaltung kann unter Verwendung von organischen Säuren, beispielsweise Trifluoressigsäure, oder wässrigen anorganischen Säuren, beispielsweise Salzsäure, oder unter Verwendung von wässrigen anorganischen Basen, beispielsweise Lithiumhydroxid, Kaliumhydroxid, Natriumhydroxid, Natriumcarbonat, Natriumhydrogencarbonat, Kaliumcarbonat in organischen Lösungsmittel, beispielsweise Methanol, Dioxan, Dichlormethan, THF, Diethylether oder in diesen Lösungsmitteln als Gemische durchgeführt werden.

Die anschließende Hydrierung kann mit Pd auf Aktivkohle, Raney Nickel, Platin oder $PtO_2$ erfolgen. Als Lösungsmittel eignen sich beispielsweise Ethanol, Methanol, Wasser, Essigsäure, Propionsäure, DCM, Cyclohexan, methanolische KOH oder Gemische dieser Lösungsmittel. Als Additiv kann beispielsweise TFA verwendet werden. Die Reaktion kann bei Normaldruck oder erhöhtem Druck durchgeführt werden. Die Reaktionszeit kann zwischen 1,5 und 72 h betragen.
Die abschließende Acylierung kann sowohl mit Säurechloriden oder -bromiden, die aus Carbonsäuren der allgemeinen Formel II hergestellt werden können, als auch mit Carbonsäuren der allgemeinen Formel II durchgeführt werden.
Die erhaltenen Carbonsäuren der allgemeinen Formel II können nach dem Fachmann bekannten Methoden in Säurechloride oder-bromide überführt werden. Die erhaltenen Carbonsäurechloride oder -bromide können in Lösungsmitteln, beispielsweise DCM, Benzol, Toluol, THF, DMF, Acetonitril, Pyridin, Dioxan, Wasser oder 1-Methyl-pyrrolidin-2-on oder Mischungen dieser Lösungsmittel, unter Verwendung von Basen, beispielsweise Pyridin, DIEA, TEA, N-Methylmorpholin oder Natriumhydrogencarbonat gegebenenfalls unter Zugabe eines Kupplungsreagenzes beispielsweise DCC, mit primären oder sekundären Aminen umgesetzt werden. Die Reaktionstemperatur kann dabei zwischen -20˚C und +110˚C variiert werden. Die Reaktionszeiten können zwischen 0,5 h und 24 h liegen.

Die Umsetzung der Carbonsäuren der allgemeinen Formel II mit primären oder sekundären Aminen kann unter Verwendung von Basen und gegebenenfalls Kupplungsreagenzien in Lösungsmitteln, beispielsweise Methanol, DMF oder DCM, durchgeführt werden. Als Basen können beispielsweise Natriummethanolat, TEA, DIEA oder N-Methylmorpholin verwendet werden. Als Kupplungsreagenzien eignen sich beispielsweise EDCI, HOBt, DCC, CDI, HBTU, DMAP oder Pentafluorphenyldiphenylphosphinat. Die Reaktionszeit kann zwischen 1 h und 3 d variieren.

**Beispiele**

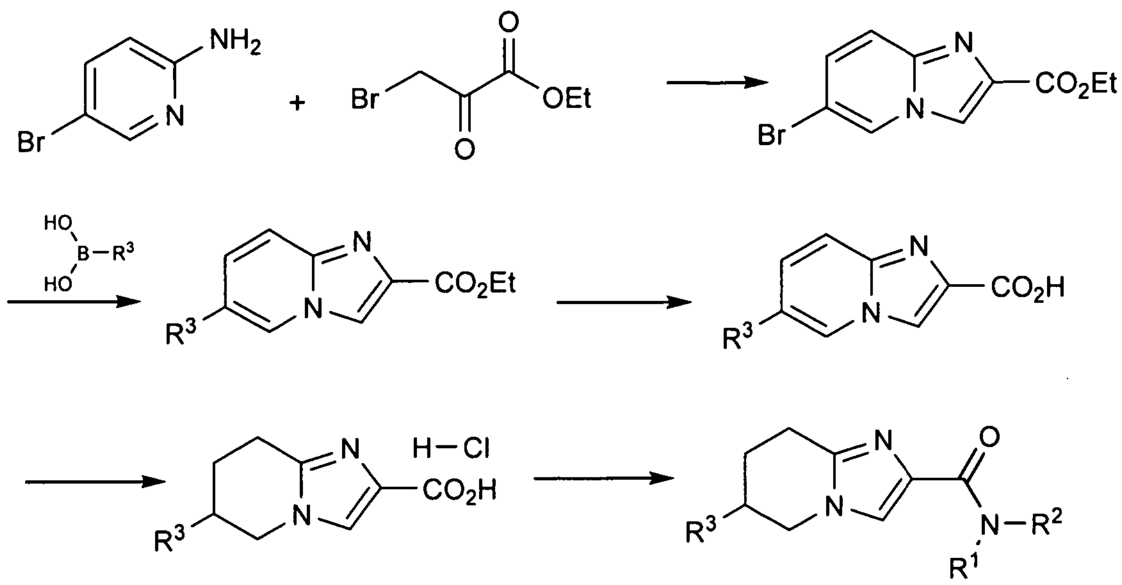

Für die Synthesen wurden folgende Tetrahydroimidazopyridine eingesetzt

| | |
|---|---|
| **S1** | 6-Phenyl-5,6,7,8-tetrahydroimidazo[1,2-a]pyridin-2-carboxylsäure |
| **S2** | 6-(4-Methoxy-3,5-dimethylphenyl)-5,6,7,8-tetrahydroimidazo[1,2-a]pyridin-2-carboxylsäure |
| **S3** | 6-(4-Fluor-3-methylphenyl)-5,6,7,8-tetrahydroimidazo[1,2-a]pyridin-2-carboxylsäure |
| **S4** | 6-(3-Methoxyphenyl)-5,6,7,8-tetrahydroimidazo[1,2-a]pyridin-2-carboxylsäure |
| **S5** | 6-(3-Fluorphenyl)-5,6,7,8-tetrahydroimidazo[1,2-a]pyridin-2-carboxylsäure |
| **S6** | 6-(4-Methoxyphenyl)-5,6,7,8-tetrahydroimidazo[1,2-a]pyridin-2-carboxylsäure |
| **S7** | 6-(5-Fluor-2-methoxyphenyl)-5,6,7,8-tetrahydroimidazo[1,2-a]pyridin-2-carboxylsäure |
| **S8** | 6-(3,5-Dimethoxyphenyl)-5,6,7,8-tetrahydroimidazo[1,2-a]pyridin-2-carboxylsäure |
| **S9** | 6-(4-Ethylphenyl)-5,6,7,8-tetrahydroimidazo[1,2-a]pyridin-2-carboxylsäure |
| **S10** | 6-(4-Fluorphenyl)-5,6,7,8-tetrahydroimidazo[1,2-a]pyridin-2-carboxylsäure |
| **S11** | 6-(4-Phenoxyphenyl)-5,6,7,8-tetrahydroimidazo[1,2-a]pyridin-2-carboxylsäure |
| **S12** | 6-(3-Methylphenyl)-5,6,7,8-tetrahydroimidazo[1,2-a]pyridin-2-carboxylsäure |
| **S13** | 6-(2,5-Difluorphenyl)-5,6,7,8-tetrahydroimidazo[1,2-a]pyridin-2-carboxylsäure |
| **S14** | 6-(3,5-Bis(trifluormethyl)phenyl)-5,6,7,8-tetrahydroimidazo[1,2-a]pyridin-2-carboxylsäure |
| **S15** | 6-(3-(Trifluormethyl)phenyl)-5,6,7,8-tetrahydroimidazo[1,2-a]pyridin-2-carboxylsäure |
| **S16** | 6-2,4-Dimethoxyphenyl)-5,6,7,8-tetrahydroimidazo[1,2-a]pyridin-2-carboxylsäure |
| **S17** | 6-(2-Methylphenyl)-5,6,7,8-tetrahydroimidazo[1,2-a]pyridin-2-carboxylsäure |
| **S18** | 6-(4-Methylphenyl)-5,6,7,8-tetrahydroimidazo[1,2-a]pyridin-2-carboxylsäure |
| **S19** | 6-(3,4-Difluorphenyl)-5,6,7,8-tetrahydroimidazo[1,2-a]pyridin-2-carboxylsäure |
| **S20** | 6-(3,5-Dimethylphenyl)-5,6,7,8-tetrahydroimidazo[1,2-a]pyridin-2-carboxylsäure |
| **S21** | 6-(4-(Benzyloxy)phenyl)-5,6,7,8-tetrahydroimidazo[1,2-a]pyridin-2- carboxylsäure |

| Edukt für Baustein | Name | Anbieter |
|---|---|---|
| **S1** | Phenylboronsäure | Acros |
| **S2** | 3,5-Dimethyl-4-methoxyphenylboronsäure | Aldrich |
| **S3** | 4-Fluor-3-methylphenylboronsäure | Aldrich |
| **S4** | 3-Methoxyphenylboronsäure | Acros |
| **S5** | 3-Fluorphenylboronsäure | Acros |
| **S6** | 4-Methoxyphenylboronsäure | Acros |
| **S7** | 5-Fluor-2-methoxyphenylboronsäure | Aldrich |
| **S8** | 2,5-Dimethoxyphenylboronsäure | Acros |
| **S9** | 4-Ethylphenylboronsäure | Acros |
| **S10** | 4-Fluorphenylboronsäure | Acros |
| **S11** | 4-Phenoxyphenylboronsäure | Acros |
| **S12** | 3-Tolylboronsäure | Acros |
| **S13** | 2,5-Difluorphenylboronsäure | Acros |
| **S14** | 3,5-Bis-(trifluormethyl)phenylboronsäure | Acros |
| **S15** | 3-Trifluormethylphenylboronsäure | Acros |
| **S16** | 2,4-Dimethoxyphenylboronsäure | Acros |
| **S17** | 2-Tolylboronsäure | Acros |
| **S18** | 4-Tolylboronsäure | Acros |
| **S19** | 3,4-Difluorphenylboronsäure | Acros |
| **S20** | 3,5-Dimethylphenylboronsäure | Aldrich |
| **S21** | 4-(Benzyloxy)phenylboronsäure | Interchim |

**6-Bromo-imidazo[1,2-a]pyridin-2-carboxylsäureethylester**

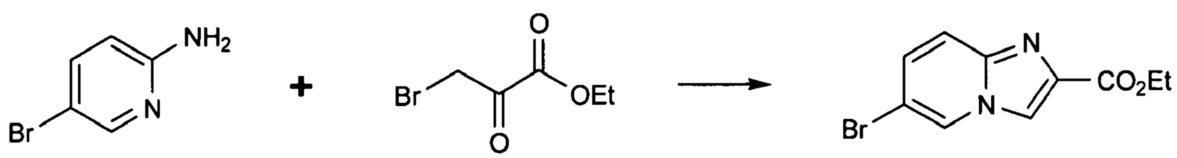

Zu einer Lösung 5-Brom-2-aminopyridin (50,0 g; 289 mmol) in ethanol (500 ml) wurde über einen Tropftrichter Ethylbromopyruvat (62,0 g; 318 mmol; 40,0 ml) zugegeben. Der Reaktionsansatz wurde zunächst 30 min. bei Raumtemperatur und anschließend 8 h unter Rückfluß gerührt (DC-Kontrolle: DCE - Ethanol 5 : 1) Das Lösungsmittel wurde im Vakuum entfernt und der Rückstand in DCM aufgenommen. Es wurde zunächst mit gesättigter Kochsalz-Lösung und anschließend mit gesättigter Natriumhydrogencarbonat-Lösung gewaschen. Die organische Phase wurde über Magnesiumsulfat getrocknet, filtriert und zur Trockne eingeengt. Der Rückstand wurde aus trockenem Diethylether umkristallisiert. Ausbeute: 41 g (53%)

**6-Phenyl-5,6,7,8-tetrahydroimidazo[1,2-a]pyridin-2-carboxylsäure S1**

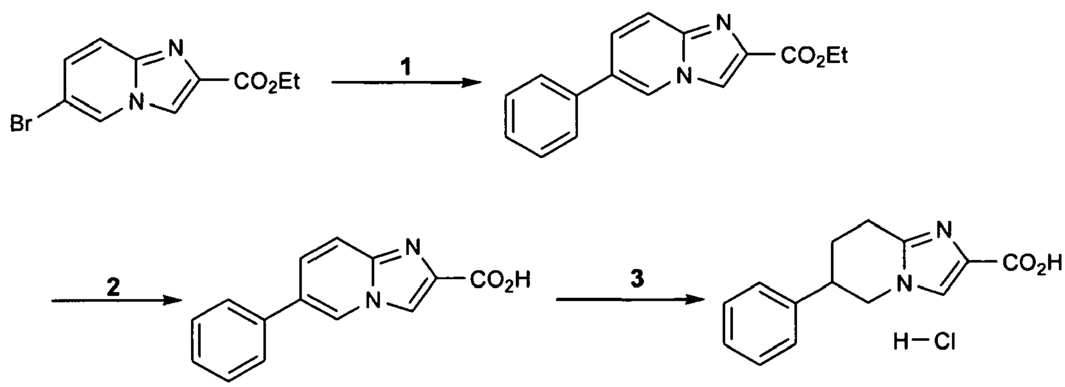


Stufe 1. Zu einer Lösung 6-Bromo-imidazo[1,2-a]pyridin-2-carboxylsäureethylester (4,71 g; 17,5 mmol) in Acetonitril (30 ml) in einem Milestone 100 ml Hochdruckgefäß wurde unter Stickstoffatmosphäre zunächst Phenylboronsäure (2,13 g; 17,5 mmol) dann KF-Lösung (2M in Wasser, 15 ml) und schließlich $Pd(PPh_3)_4$ (607 mg; 0,5 mmol; 3 mol%) gegeben. Das Reaktionsgefäß wurde mit Stickstoff gespült, verschlossen und bei 160°C für 5 min. in der Mikrowelle bestrahlt. (DC-Kontrolle: n-Hexan-Ethylacetat 1:1).

Es wurden 2 Ansätze der gleichen Ansatzmenge parallel durchgeführt und vor der Aufarbeitung vereinigt.

Das ausgefallene Produkt wurde abfiltriert und mit Diethylether gewaschen. Das schwarze kristalline Produkt wurde in Chloroform aufgenommen und erst über Kieselgel und dann über Celite filtriert. Das klare Filtrat wurde bis zur Trockne eingeengt.

Ausbeute: 9,3 g (100 %)

Stufe 2. Der 6-Phenyl-imidazo[1,2-a]pyridine-2-carboxysäureethylester (9,32 g, 17,5 mmol) wurde in Ethanol (80 ml) gelöst und mit NaOH-Lösung (20% in Wasser, 7 ml) versetzt. Der Reaktionsansatz wurde 8 h unter Rückfluß erhitzt. Nach beendeter Reaktion wurde das Lösungsmittel am Rotationsverdampfer entfernt und der Rückstand in einer kleinen Menge Wasser aufgenommen. Die Lösung wurde vorsichtig mit HCl-Lösung (10% in Wasser) auf pH 3-4 eingestellt. Das ausgefallene Produkt wurde filtriert und mit Wasser gewaschen und ohne weitere Aufreinigung in die nächste Stufe eingesetzt.

Ausbeute: 7,90 g (95%)

Stufe 3. Zu einer Lösung der 6-Phenyl-imidazo[1,2-a]pyridin-2-carboxylsäure (6,3 g; 26,4 mmol) in einer Mischung aus HCl (4% in Wasser, 100 ml) und Ethanol (200 ml) wurde Palladium auf Aktivkohle (1,5 g) gegeben. Die Mischung wurde in einen Autoklaven (stainless steel) geben, der Autoklav wurde verschlossen und einige Male mit Stickstoff gespült. Der Autoklav wurde mit Wasserstoff gefüllt (8 bar) und der Reaktionsansatz über das Wochenende bei Raumtemperatur gerührt. Der Katalysator über Celite abfiltriert und das Lösungsmittel am Rotationsverdampfer bei Raumtemperatur abgezogen. Das Produkt wurde aus Acetonitril kristallisiert. Ausbeute: 4,1g (56%)

**6-(4-Methoxy-3,5-dimethylphenyl)-5,6,7,8-tetrahydroimidazo[1,2-a]pyridin-2-carboxylsäure Hydrochlorid S2**

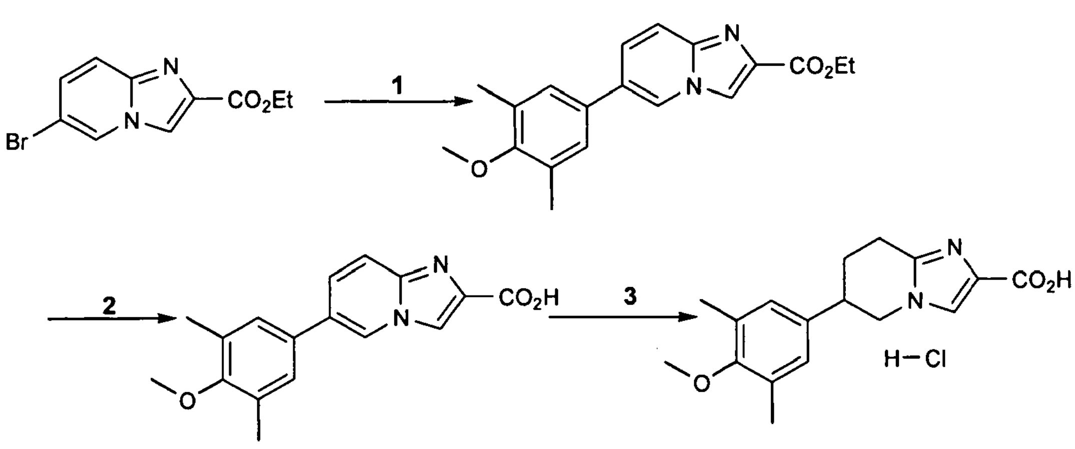

Stufe 1. Zu einer Lösung 6-Bromo-imidazo[1,2-a]pyridin-2-carboxylsäureethylester (4,71 g; 17,5 mmol) in Acetonitril (30 ml) in einem Milestone 100 ml Hochdruckgefäß wurde unter Stickstoffatmosphäre zunächst 4-Methoxy-3,5-dimethylphenylboronsäure (3,15 g; 17,5 mmol) dann KF-Lösung (2M in Wasser, 15 ml) und schließlich $Pd(PPh_3)_4$ (607 mg; 0,525 mmol; 3 mol%) gegeben. Das Reaktionsgefäß wurde mit Stickstoff gespült, verschlossen und bei 160°C für 5 min. in der Mikrowelle bestrahlt. (DC-Kontrolle: n-Hexan-Ethylacetat 1:1).
Es wurden 4 Ansätze der gleichen Ansatzmenge parallel durchgeführt und vor der Aufarbeitung vereinigt.
Das ausgefallene Produkt wurde abfiltriert und mit Diethylether gewaschen. Das schwarze kristalline Produkt wurde in Chloroform aufgenommen und über Celite filtriert. Das klare Filtrat wurde eingeengt und ohne weitere Aufreinigung für die weitere Umsetzung verwendet.
Ausbeute: 22,7g (quant.)
Stufe 2. Der 6-(4-Methoxy-3,5-dimethyl-phenyl)-imidazo[1,2-a]pyridin-2-carboxylsäureethyl-ester (22,7 g, 70 mmol) wurde in Ethanol (300 ml) gelöst und mit NaOH-Lösung (20% in Wasser, 30 ml) versetzt. Der Reaktionsansatz wurde 24 h unter Rückfluß erhitzt. Nach beendeter Reaktion wurde das Lösungsmittel am Rotationsverdampfer entfernt und der Rückstand in einer möglichst kleinen Menge Wasser aufgenommen. Die Lösung wurde vorsichtig mit HCl-Lösung (10% in Wasser) auf pH 3-4 eingestellt. Das ausgefallene Produkt wurde filtriert und mit Wasser gewaschen.
Ausbeute: 21,2 (quant.)
Stufe 3. Zu einer Lösung der 6-(4-Methoxy-3,5-dimethylphenyl)-imidazo[1,2-a]pyridin-2-carboxylsäure (4,74 g; 16 mmol) in einer Mischung aus HCl (4% in Wasser, 70 ml) und Ethanol (140 ml) wurde Palladium auf Aktivkohle (1 g) gegeben. Die Mischung wurde in einen Autoklaven (stainless steel) geben, der Autoklav wurde verschlossen und einige Male mit Stickstoff gespült. Der Autoklav wurde mit Wasserstoff gefüllt (8 bar) und der Reaktionsansatz für 12 h bei Raumtemperatur gerührt.
Nach beendeter Reaktion wurde der Katalysator über Celite abfiltriert und das Lösungsmittel am Rotationsverdampfer bei Raumtemperatur abgezogen. Das Produkt wurde aus Acetonitril kristallisiert.
Ausbeute: 2,3 g (43%)

**6-(4-Fluor-3-methylphenyl)-5,6,7,8-tetrahydroimidazo[1,2-a]pyridin-2-carboxylsäure S3**

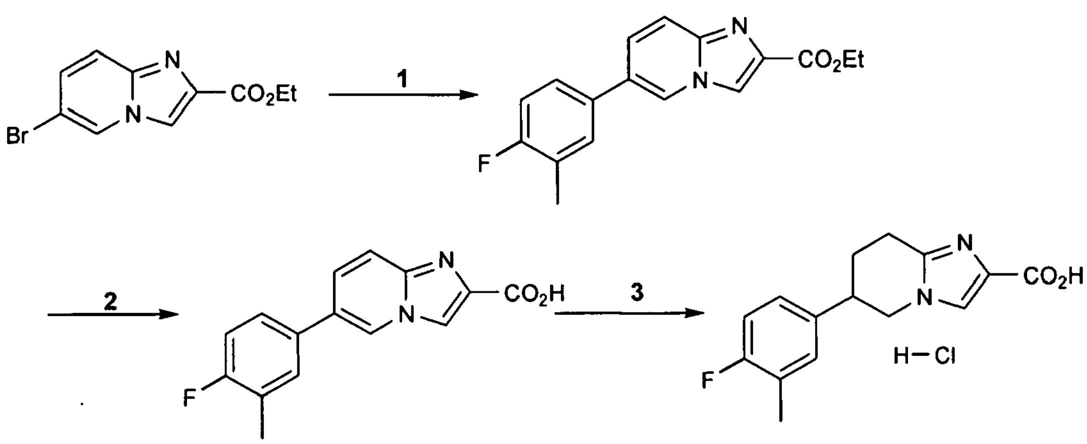


Stufe 1. Zu einer Lösung 6-Bromo-imidazo[1,2-a]pyridin-2-carboxylsäureethylester (4,04 g; 15 mmol) in Acetonitril (30 ml) in einem Milestone 100 ml Hochdruckgefäß wurde unter Stickstoffatmosphäre zunächst 4-Fluor-3-methylphenylboronsäure (4,62 g; 15 mmol) dann KF-Lösung (2M in Wasser, 15 ml) und schließlich $Pd(PPh_3)_4$ (520 mg; 0,45 mmol; 3 mol%) gegeben. Das Reaktionsgefäß wurde mit Stickstoff gespült, verschlossen und bei 160°C für 5 min. in der Mikrowelle bestrahlt. (DC-Kontrolle: n-Hexan-Ethylacetat 1:1).
Es wurden 2 Ansätze der gleichen Ansatzmenge parallel durchgeführt und vor der Aufarbeitung vereinigt.
Das ausgefallene Produkt wurde abfiltriert und mit Diethylether gewaschen. Das schwarze kristalline Produkt wurde in Chloroform aufgenommen und über Celite filtriert. Das klare Filtrat wurde eingeengt und aus Diethylether kristallisiert.
Ausbeute: 4,5 g (50 %)
Stufe 2. Der 6-(4-Fluor-3-methyl-phenyl)-imidazo[1,2-a]pyridin-2-carboxylsäureethylester (3,0 g, 10,1 mmol) wurde in Ethanol (50 ml) gelöst und mit NaOH-Lösung (20% in Wasser, 4 ml) versetzt. Der Reaktionsansatz wurde 2 h unter Rückfluß erhitzt. Nach beendeter Reaktion wurde das Lösungsmittel am Rotationsverdampfer entfernt und der Rückstand in einer möglichst kleinen Menge Wasser aufgenommen. Die Lösung wurde vorsichtig mit HCl-Lösung (10% in Wasser)

auf pH 3-4 eingestellt. Das ausgefallene Produkt wurde filtriert und mit Wasser gewaschen. Das Rohprodukt wurde ohne weitere Aufreinigung in die nächste Stufe eingesetzt.
Ausbeute: 1,69 g (62 %)
Stufe 3. Zu einer Lösung der 6-(4-Fluor-3-methyl-phenyl)-imidazo[1,2-a]pyridin-2-carboxylsäure (1,69 g; 6,2 mmol) in einer Mischung aus HCl (4% in Wasser, 20 ml) und Ethanol (40 ml) wurde Palladium auf Aktivkohle (350 mg) gegeben. Die Mischung wurde in einen Autoklaven (stainless steel) geben, der Autoklav wurde verschlossen und einige Male mit Stickstoff gespült. Der Autoklav wurde mit
Wasserstoff gefüllt (8 bar) und der Reaktionsansatz für 20 h bei Raumtemperatur gerührt.
Der Katalysator wurde über Celite abfiltriert und das Lösungsmittel am Rotationsverdampfer bei Raumtemperatur abgezogen. Das Produkt wurde aus Acetonitril kristallisiert.
Da die Reaktion nicht vollständig verlief wurde das Rohprodukt-Gemisch erneut in einer Mischung aus HCl (4% in Wasser, 15 ml) und Ethanol (30 ml) gelöst und mit Palladium auf Aktivkohle (260 mg) versetzt, in einen Autoklaven (stainless steel) geben, verschlossen und einige Male mit Stickstoff gespült. Der Autoklav wurde mit Wasserstoff gefüllt (8 bar) und der Reaktionsansatz für weitere 12 h bei
Raumtemperatur gerührt. Der Katalysator wurde über Celite abfiltriert und das Lösungsmittel am Rotationsverdampfer bei Raumtemperatur abgezogen. Das Produkt wurde aus Acetonitril kristallisiert.
Ausbeute: 609 mg (31 %)

**6-(3-Methoxy-phenyl)-5,6,7,8-tetrahydro-imidazo[1,2-a]pyridin-2-carboxylsäure Hydrochlorid S4**

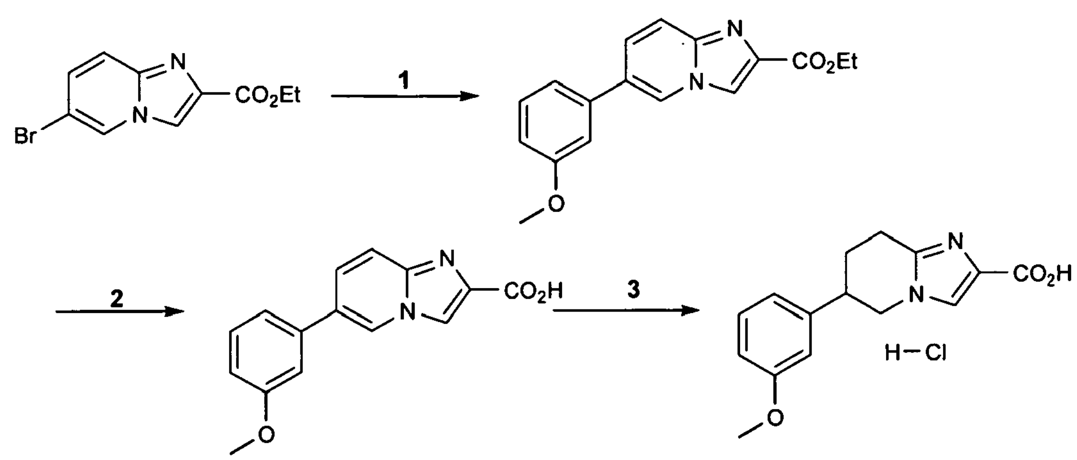


Stufe 1. Zu einer Lösung 6-Bromo-imidazo[1,2-a]pyridin-2-carboxylsäureethylester (4,71 g; 17,5 mmol) in Acetonitril (30 ml) in einem Milestone 100 ml Hochdruckgefäß wurde unter Stickstoffatmosphäre zunächst 3-Methoxyphenylboronsäure (2,66 g; 17,5 mmol) dann KF-Lösung (2M in Wasser, 15 ml) und schließlich $Pd(PPh_3)_4$ (606 mg; 0,525 mmol; 3 mol%) gegeben. Das Reaktionsgefäß wurde mit Stickstoff gespült, verschlossen und bei 160˚C für 5 min. in der Mikrowelle bestrahlt. (DC-Kontrolle: *n*-Hexan-Ethylacetat 1:1).
Es wurden 4 Ansätze der gleichen Ansatzmenge parallel durchgeführt und vor der Aufarbeitung vereinigt.
Das ausgefallene Produkt wurde abfiltriert und mit Diethylether gewaschen. Das schwarze kristalline Produkt wurde in Chloroform aufgenommen und über Celite filtriert. Das klare Filtrat wurde eingeengt und aus Diethylether kristallisiert.
Ausbeute: 15,48 g (75%)
Stufe 2. Der 6-(3-Methoxy-phenyl)-imidazo[1,2-a]pyridin-2-carboxylsäureethylester (15,48 g, 52 mmol) wurde in Ethanol (150 ml) gelöst und mit NaOH-Lösung (20% in Wasser, 10 ml) versetzt. Der Reaktionsansatz wurde 8 h unter Rückfluß erhitzt. Nach beendeter Reaktion wurde das Lösungsmittel am Rotationsverdampfer entfernt und der Rückstand in einer möglichst kleinen Menge Wasser aufgenommen. Die Lösung wurde vorsichtig mit HCl-Lösung (10% in Wasser) auf pH 3-4 eingestellt. Das ausgefallene Produkt wurde filtriert und mit Wasser gewaschen.
Ausbeute: 7,93 g (57%)
Stufe 3. Zu einer Lösung der 6-(3-Methoxy-phenyl)-imidazo[1,2-a]pyridin-2-carboxylsäure (1,38 g; 5,15 mmol) in einer Mischung aus HCl (4% in Wasser, 60 ml) und Ethanol (90 ml) wurde Palladium auf Aktivkohle (300 mg) gegeben. Die Mischung wurde in einen Autoklaven (stainless steel) geben, der Autoklav wurde verschlossen und einige Male mit Stickstoff gespült. Der Autoklav wurde mit Wasserstoff gefüllt (8 bar) und der Reaktionsansatz für 5 h bei Raumtemperatur gerührt. Nach beendeter Reaktion wurde der Katalysator über Celite abfiltriert und das Lösungsmittel am Rotationsverdampfer bei Raumtemperatur abgezogen. Das Produkt wurde aus Acetonitril kristallisiert.
Ausbeute: 1,30 g (82%)

**Synthese der Bausteine S5, S6, S10, S12, S18 - S20**

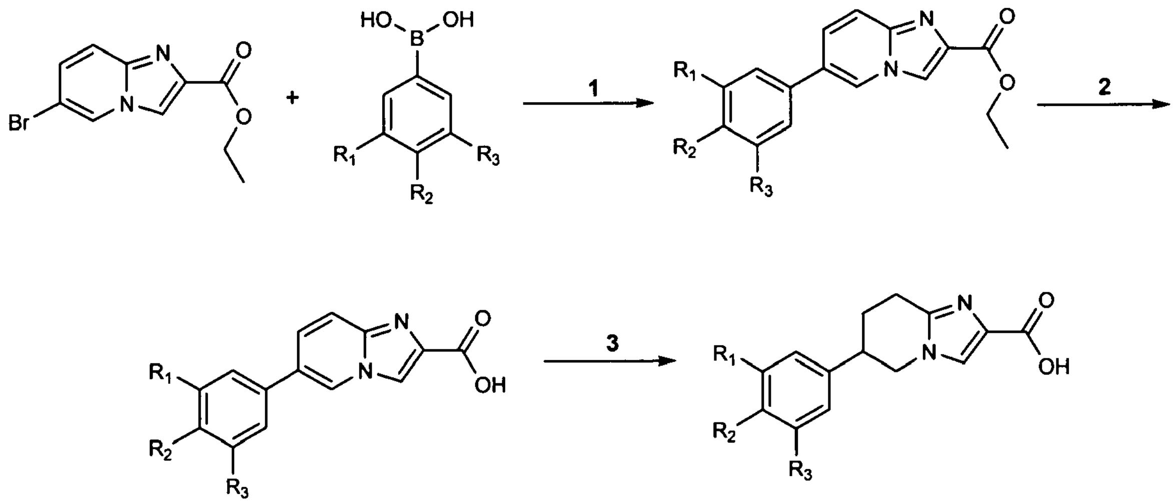


Stufe 1. Zu einer Lösung 6-Bromo-imidazo[1,2-a]pyridin-2-carboxylsäureethylester (1,86 mmol) in Toluol (7 ml) wurde zunächst die entsprechende Boronsäure (2,04 mmol) dann $K_2CO_3$ (4,65 mmol), Wasser (1,5 ml) und Ethanol (3,5 ml) gegeben. Durch die Reaktionslösung wurde 15 min. Argon geleitet. Zuletzt wurde $Pd(PPh_3)_4$ (0,093 mmol) zugegeben und weitere 15 min. mit Argon begast. Anschließend wurde 15 h unter Rückfluß erhitzt. Nach abgeschlossener Reaktion (DC-Kontrolle) wurde der Reaktionsansatz mit Ethylacetat verdünnt und erst mit Wasser und anschließend mit ges. Natriumchlorid-Lösung gewaschen. Die organische Phase wurde über Natriumsulfat getrocknet und das Lösungsmittel vollständig abgezogen. Die Produkte wurden aus einer Mischung aus 25 % Diethylether in Hexan kristallisiert, filtriert und getrocknet.
Stufe 2. Das Suzuki-Produkt wurde in Ethanol gelöst und mit NaOH-Lösung (20% in Wasser, versetzt. Der Reaktionsansatz wurde 2 h unter Rückfluß erhitzt. Nach beendeter Reaktion wurde das Lösungsmittel am Rotationsverdampfer entfernt und der Rückstand in einer möglichst kleinen Menge Wasser aufgenommen. Die Lösung wurde vorsichtig mit HCl-Lösung (10% in Wasser) auf pH 3-4 eingestellt. Das ausgefallene Produkt wurde filtriert und mit Wasser gewaschen. Das Produkt wurde in Toluol aufgenommen, eingeengt und anschließend getrocknet.
Stufe 3. Zu einer Lösung der freien Säure in einer Mischung aus HCl (3% in Wasser, 175 ml) und Ethanol (88 ml) wurde Palladium auf Aktivkohle (1,53 g) gegeben. Die Mischung wurde in einen Autoklaven (stainless steel) geben, der Autoklav wurde verschlossen und einige Male mit Stickstoff gespült. Der Autoklav wurde mit
Wasserstoff gefüllt (8 bar) und der Reaktionsansatz für 8 h bei Raumtemperatur gerührt. Nach beendeter Reaktion wurde der Katalysator über Celite abfiltriert und das Lösungsmittel am Rotationsverdampfer bei 45°C abgezogen. Das Produkt wurde aus Methanol-Diethylether kristallisiert.

| | $R_1$ | $R_2$ | $R_3$ | Ausbeute (über 3 Stufen (%) |
|---|---|---|---|---|
| **S5** | F | H | H | 25 |
| **S6** | H | $OCH_3$ | H | 18 |
| **S10** | H | F | H | 10 |
| **S12** | $CH_3$ | H | H | 36 |
| **S18** | H | $CH_3$ | H | 21 |
| **S19** | F | F | H | 33 |
| **S20** | $CH_3$ | H | $CH_3$ | 61 |

Für die Synthesen wurden die folgenden kommerziell erhältlichen Amine verwendet:

| Nr. | Name | Anbieter |
|---|---|---|
| **A01** | 1-(2-Methoxyphenyl)piperazin | Aldrich |
| **A02** | Benzylamin | Aldrich |
| **A03** | 2-Phenylethylamin | Aldrich |
| **A04** | 3-Picolylamin | Aldrich |
| **A05** | 4-Benzylpiperidin | Aldrich |
| **A06** | Cyclopropylamin | Aldrich |
| **A07** | Ethyl piperidin-4-carboxylat | Aldrich |
| **A08** | N-Benzyl-2-phenethylamin | Aldrich |
| **A09** | 2-(3,4-Dimethoxyphenyl)ethanamin | Aldrich |
| **A10** | Ethyl piperidin-3-carboxylat | Aldrich |
| **A11** | 1-(Piperazin-1-yl)ethanon | Aldrich |
| **A12** | Pyrrolidin | Aldrich |
| **A13** | 4-(2-Fluorphenyl)-piperazin | Aldrich |
| **A14** | Benzo[d][1,3]dioxol-5-ylmethanamin | Aldrich |
| **A15** | Butylamin | Aldrich |
| **A16** | 4-Fluorbenzylamin | Aldrich |
| **A17** | 1,3,3-Trimethyl-6-azabicyclo[3.2.1]octan | Acros |
| **A18** | Amylamin | Aldrich |
| **A19** | 4-(3-Trifluormethylphenyl)-piperazin | Aldrich |
| **A20** | Hexylamin | Aldrich |
| **A21** | 4-Methylpiperidin | Aldrich |
| **A22** | 4-Phenyl-butyl-2-amin | Aldrich |
| **A23** | (3-(Trifluoromethyl)phenyl)methanamin | Aldrich |
| **A24** | sec.-Butylamin | Aldrich |
| **A25** | 4-(4-Methoxyphenyl)-piperazin | Aldrich |
| **A26** | a-Methylbenzylamin | Aldrich |
| **A27** | 4-(2-Ethoxyphenyl)-piperazin | Aldrich |
| **A28** | 1-Benzhydrylpiperazin | Acros |
| **A29** | 1-(4-(Trifluoromethyl)phenyl)piperazin | Acros |
| **A30** | 4-(4-Fluorphenyl)-piperazin | Acros |
| **A31** | 4-(4-Methylphenyl)-piperazin | Acros |
| **A32** | 4-(3-Methylphenyl)-piperazin | Acros |
| **A33** | Cyclohexylmethanamin | Lancaster |
| **A34** | 1-Butylpiperazin | Fluka |
| **A36** | 1-Benzylpiperazin | ACBBlocks |

**Synthese des 1-(3,5-Dimethylphenyl)ethanamin A35**

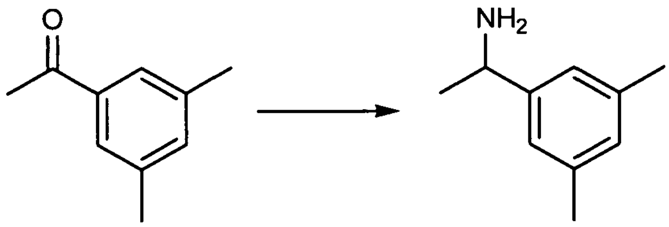

3,5-Dimethylacetophenon (3,0 g, 20,2 mmol) wurden in ethanolischer $NH_3$-Lösung (2 M in Ethanol, 50 ml) gelöst und mit Tetrapropylortotitanat (11 ml, 40,4 ml) versetzt und 6 h bei Raumtemperatur gerührt. Anschließend wurde die Reaktionslösung vorsichtig mit $NaBH_4$ (1,16 g, 30,4 mmol) versetzt und über Nacht bei Raumtemperatur gerührt.
Das Reaktionsgemisch wurde in wässrige Ammoniaklösung gegossen und filtriert.
Der Feststoff wurde noch zweimal mit Ethylacetat (50 ml) gewaschen. Die Phasen wurden getrennt und die wäßrige Phase noch zweimal mit Ethylacetat extrahiert. Die vereinigten organischen Phasen wurden mit HCl-Lösung (2 N, 60 ml) gewaschen, die wäßrige Phase mit NaOH-Lösung auf pH11 eingestellt und dreimal mit Ethylacetat (100 ml) extrahiert. Die organische Phase wurde mit ges. NaCl-Lösung (100 ml) gewaschen, über $MgSO_4$ getrocknet und eingeengt.
Das erhaltene Rohprodukt wurde ohne weitere Aufreinigung weiter eingesetzt.
Ausbeute: 43 %

**Beispielverbindungen**

**Automatisierte Synthese**

Herstellung der Stammlösungen:
Lösung A (Lösung des Säurebausteins in DMA):
Das verwendete Hydrochlorid der Säure wurde vor dem Gebrauch zunächst in einem Vakuum Trockenschrank getrocknet. Die entsprechende Verbindung wurde zunächst mit 3 Mol-Äquivalenten 4-Methylmorpholin in DMA bei Raumtemperatur gemischt. Anschließend wurde soviel Lösungsmittel zugegeben, dass eine 0,15-0,2 molare
Lösung entstand.

Lösung B (Lösung TBTU in DMA)
Die Lösung B wurde aus TBTU in DMA hergestellt (0,3 mol/l)

Lösung C (Lösung des Amin-Bausteins in DMA)
Die Lösung C wurde aus dem entsprechenden Amin in DMA hergestellt (0,5 mol/l)

Lösung A (100 $\mu$mol, 0,5 - 0,66 ml) und Lösung B (120 $\mu$mol, 0,4 ml) wurden mit Hilfe eines Cavro RSP9000 Robotic Systems in Standard Glas-Reaktionsgefäße gegeben und 15 Min. reagiert. Anschließend wurde die Lösung C (120 $\mu$mol, 0,24 ml) mit Hilfe eines Cavro RSP9000 Robotic Systems zugegeben. Die Reaktionsansätze wurden 16-20 h bei Raumtemperatur gerührt (DC-Kontrolle)
Zur Aufarbeitung wurde zunächst das Lösungsmittel im Vakuum entfernt und anschließend wurde der Rückstand mit Chloroform (2,5 ml) versetzt. Die Lösung wurde anschließend erst mit Wasser (1 ml), dann mit NaOH-Lösung (10% in Wasser, 1 ml) und anschließend mit Wasser (1 ml) extrahiert.
Das Lösungsmittel wurde vollständig entfernt. Das Produkt wurde mit HPLC-MS analysiert und bei einer Reinheit von < 85% durch präparative HPLC aufgereinigt.

Es wurden die folgenden Verbindungen durch automatisierte Synthese hergestellt.

| Nr. | Edukt S | Edukte A | Name | MS m/z $[M+H]^+$ |
|---|---|---|---|---|
| **1** | S1 | A05 | (4-Benzylpiperidin-1-yl)(6-phenyl-5,6,7,8-tetrahydroimidazo[1,2-a]pyridin-2-yl)methanon | 400,2 |
| **2** | S1 | A08 | N-Benzyl-N-phenethyl-6-phenyl-5,6,7,8-tetrahydroimidazo[1,2-a]pyridin-2-carboxamid | 436,2 |
| **3** | S1 | A17 | (6-Phenyl-5,6,7,8-tetrahydroimidazo[1,2-a]pyridin-2-yl)(1,3,3-trimethyl-6-azabicyclo[3.2.1]octan-6-yl)methanon | 378,2 |

(fortgesetzt)

| Nr. | Edukt S | Edukte A | Name | MS m/z [M+H]$^+$ |
|---|---|---|---|---|
| **4** | S5 | A17 | (6-(3-Fluorphenyl)-5,6,7,8-tetrahydroimidazo[1,2-a]pyridin-2-yl)(1,3,3-trimethyl-6-azabicyclo[3.2.1]octan-6-yl)methanon | 396,2 |
| **5** | S2 | A02 | N-Benzyl-6-(4-methoxy-3,5-dimethylphenyl)-5,6,7,8-tetrahydroimidazo[1,2-a]pyridin-2-carboxamid | 390,2 |
| **6** | S2 | A13 | (4-(2-Fluorphenyl)piperazin-1-yl)(6-(4-methoxy-3,5-dimethylphenyl)-5,6,7,8-tetrahydroimidazo[1,2-a]pyridin-2-yl) methanon | 463,2 |
| **7** | S7 | A19 | (6-(5-Fluor-2-methoxyphenyl)-5,6,7,8-tetrahydroimidazo[1,2-a] pyridin-2-yl)(4-(3-(trifluormethyl)phenyl)piperazin-1-yl)methanon | 503,2 |
| **8** | S7 | A05 | (4-Benzylpiperidin-1-yl)(6-(5-fluor-2-methoxyphenyl)-5,6,7,8-tetrahydroimidazo[1,2-a]pyridin-2-yl)methanon | 448,2 |
| **9** | S7 | A17 | (6-(5-Fluor-2-methoxyphenyl)-5,6,7,8-tetrahydroimidazo[1,2-a] pyridin-2-yl)(1,3,3-trimethyl-6-azabicyclo[3.2.1]octan-6-yl) methanon | 426,2 |
| **10** | S2 | A01 | (6-(4-Methoxy-3,5-dimethylphenyl)-5,6,7,8-tetrahydroimidazo [1,2-a]pyridin-2-yl)(4-(2-Methoxyphenyl)piperazin-1-yl)methanon | 475,3 |
| **11** | S9 | A05 | (4-Benzylpiperidin-1-yl)(6-(4-ethylphenyl)-5,6,7,8-tetrahydroimidazo[1,2-a]pyridin-2-yl)methanon | 428,3 |
| **12** | S8 | A17 | (6-(2,5-Dimethoxyphenyl)-5,6,7,8-tetrahydroimidazo[1,2-a] pyridin-2-yl)(1,3,3-trimethyl-6-azabicyclo[3.2.1 ]octan-6-yl) methanon | 438,3 |
| **13** | S3 | A13 | (6-(4-Fluor-3-methylphenyl)-5,6,7,8-tetrahydroimidazo[1,2-a] pyridin-2-yl)(4-(2-Fluorphenyl)piperazin-1-yl)methanon | 437,2 |
| **14** | S3 | A26 | 6-(4-Fluor-3-methylphenyl)-N-(1-phenylethyl)-5,6,7,8-tetrahydroimidazo[1,2-a]pyridin-2-carboxamid | 378,2 |
| **15** | S2 | A18 | 6-(4-Methoxy-3,5-dimethylphenyl)-N-pentyl-5,6,7,8-tetrahydroimidazo[1,2-a]pyridin-2-carboxamid | 370,2 |
| **16** | S2 | A20 | N-Hexyl-6-(4-methoxy-3,5-dimethylphenyl)-5,6,7,8-tetrahydroimidazo[1,2-a]pyridin-2-carboxamid | 384,3 |
| **17** | S2 | A23 | 6-(4-Methoxy-3,5-dimethylphenyl)-N-(3-(trifluormethyl)Benzyl)-5,6,7,8-tetrahydroimidazo[1,2-a]pyridin-2-carboxamid | 458,2 |
| **18** | S11 | A24 | N-sec-Butyl-6-(4-phenoxyphenyl)-5,6,7,8-tetrahydroimidazo[1,2-a]pyridin-2-carboxamid | 390,2 |
| **19** | S11 | A15 | N-Butyl-6-(4-phenoxyphenyl)-5,6,7,8-tetrahydroimidazo[1,2-a] pyridin-2-carboxamid | 390,2 |
| **20** | S11 | A07 | Ethyl 1-(6-(4-phenoxyphenyl)-5,6,7,8-tetrahydroimidazo[1,2-a] pyridin-2-carbonyl)piperidin-4-carboxylat | 474,2 |
| **21** | S11 | A21 | (4-Methylpiperidin-1-yl)(6-(4-phenoxyphenyl)-5,6,7,8-tetrahydroimidazo[1,2-a]pyridin-2-yl)methanon | 416,2 |
| **22** | S11 | A10 | Ethyl 1-(6-(4-phenoxyphenyl)-5,6,7,8-tetrahydroimidazo[1,2-a] pyridin-2-carbonyl)piperidine-3-carboxylate | 474,2 |
| **23** | S11 | A11 | 1-(4-(6-(4-Phenoxyphenyl)-5,6,7,8-tetrahydroimidazo[1,2-a] pyridin-2-carbonyl)piperazin-1-yl)ethanon | 445,2 |
| **24** | S11 | A04 | 6-(4-Phenoxyphenyl)-N-(pyridin-3-ylmethyl)-5,6,7,8-tetrahydroimidazo[1,2-a]pyridin-2-carboxamid | 425,2 |
| **25** | S11 | A12 | (6-(4-Phenoxyphenyl)-5,6,7,8-tetrahydroimidazo[1,2-a]pyridin-2-yl)(pyrrolidin-1-yl)methanon | 388,2 |
| **26** | S11 | A06 | N-Cyclopropyl-6-(4-phenoxyphenyl)-5,6,7,8-tetrahydroimidazo [1,2-a]pyridin-2-carboxamid | 374,2 |
| **27** | S11 | A09 | N-(3,4-Dimethoxyphenethyl)-6-(4-phenoxyphenyl)-5,6,7,8-tetrahydroimidazo[1,2-a]pyridin-2-carboxamid | 498,2 |

(fortgesetzt)

| Nr. | Edukt S | Edukte A | Name | MS m/z [M+H]$^+$ |
|---|---|---|---|---|
| **28** | S11 | A18 | N-Pentyl-6-(4-phenoxyphenyl)-5,6,7,8-tetrahydroimidazo[1,2-a] pyridin-2-carboxamid | 404,2 |
| **29** | S12 | A12 | Pyrrolidin-1-yl(6-m-tolyl-5,6,7,8-tetrahydroimidazo[1,2-a]pyridin-2-yl)methanon | 310,2 |
| **30** | S14 | A12 | (6-(3,5-Bis(trifluormethyl)phenyl)-5,6,7,8-tetrahydroimidazo[1,2-a]pyridin-2-yl)(pyrrolidin-1-yl)methanon | 432,1 |
| **31** | S15 | A12 | Pyrrolidin-1-yl(6-(3-(trifluormethyl)phenyl)-5,6,7,8-tetrahydroimidazo[1,2-a]pyridin-2-yl)methanon | 364,2 |
| **32** | S15 | A13 | (4-(2-Fluorphenyl)piperazin-1-yl)(6-(3-(triFluormethyl)phenyl)-5,6,7,8-tetrahydroimidazo[1,2-a]pyridin-2-yl)methanon | 473,2 |
| **33** | S16 | A12 | (6-(2,4-Dimethoxyphenyl)-5,6,7,8-tetrahydroimidazo[1,2-a] pyridin-2-yl)(pyrrolidin-1-yl)methanon | 356,2 |
| **34** | S17 | A12 | Pyrrolidin-1-yl(6-o-tolyl-5,6,7,8-tetrahydroimidazo[1,2-a]pyridin-2-yl)methanon | 310,2 |
| **35** | S17 | A19 | (6-o-Tolyl-5,6,7,8-tetrahydroimidazo[1,2-a]pyridin-2-yl) (4-(3-(trifluormethyl)phenyl)piperazin-1-yl)methanon | 469,2 |
| **36** | S13 | A12 | (6-(2,5-Difluorphenyl)-5,6,7,8-tetrahydroimidazo[1,2-a]pyridin-2-yl)(pyrrolidin-1-yl)methanon | 332,1 |
| **37** | S9 | A12 | (6-(4-Ethylphenyl)-5,6,7,8-tetrahydroimidazo[1,2-a]pyridin-2-yl) (pyrrolidin-1-yl)methanon | 324,2 |
| **38** | S10 | A17 | (6-(4-Fluorphenyl)-5,6,7,8-tetrahydroimidazo[1,2-a]pyridin-2-yl) (1,3,3-trimethyl-6-azabicyclo[3.2.1]octan-6-yl)methanon | 396,2 |
| **39** | S6 | A23 | 6-(4-Methoxyphenyl)-N-(3-(trifluormethyl)benzyl)-5,6,7,8-tetrahydroimidazo[1,2-a]pyridin-2-carboxamid | 430,2 |
| **40** | S9 | A03 | 6-(4-Ethylphenyl)-N-phenEthyl-5,6,7,8-tetrahydroimidazo[1,2-a] pyridin-2-carboxamid | 374,2 |
| **41** | S10 | A05 | (4-Benzylpiperidin-1-yl)(6-(4-fluorphenyl)-5,6,7,8-tetrahydroimidazo[1,2-a]pyridin-2-yl)methanon | 418,2 |
| **42** | S14 | A14 | N-(Benzo[d][1,3]dioxol-5-ylmethyl)-6-(3,5-bis(trifluormethyl) phenyl)-5,6,7,8-tetrahydroimidazo[1,2-a]pyridin-2-carboxamid | 512,1 |
| **43** | S14 | A02 | N-Benzyl-6-(3,5-bis(trifluormethyl)phenyl)-5,6,7,8-tetrahydroimidazo[1,2-a]pyridin-2-carboxamid | 468,1 |
| **44** | S2 | A25 | (6-(4-Methoxy-3,5-dimethylphenyl)-5,6,7,8-tetrahydroimidazo [1,2-a]pyridin-2-yl)(4-(4-Methoxyphenyl)piperazin-1-yl)methanon | 475,3 |
| **45** | S12 | A27 | (4-(2-Ethoxyphenyl)piperazin-1-yl)(6-m-tolyl-5,6,7,8-tetrahydroimidazo[1,2-a]pyridin-2-yl)methanon | 445,3 |
| **46** | S16 | A19 | (6-(2,4-Dimethoxyphenyl)-5,6,7,8-tetrahydroimidazo[1,2-a] pyridin-2-yl)(4-(3-(trifluormethyl)phenyl)piperazin-1-yl)methanon | 515,2 |
| **47** | S10 | A19 | (6-(4-Fluorphenyl)-5,6,7,8-tetrahydroimidazo[1,2-a]pyridin-2-yl) (4-(3-(triFluormethyl)phenyl)piperazin-1-yl)methanon | 473,2 |
| **48** | S5 | A19 | (6-(3-Fluorphenyl)-5,6,7,8-tetrahydroimidazo[1,2-a]pyridin-2-yl) (4-(3-(trifluormethyl)phenyl)piperazin-1-yl)methanon | 473,2 |
| **49** | S2 | A16 | N-(4-Fluorbenzyl)-6-(4-methoxy-3,5-dimethylphenyl)-5,6,7,8-tetrahydroimidazo[1,2-a]pyridin-2-carboxamid | 408,2 |
| **50** | S4 | A05 | (4-Benzylpiperidin-1-yl)(6-(3-methoxyphenyl)-5,6,7,8-tetrahydroimidazo[1,2-a]pyridin-2-yl)methanon | 430,2 |
| **51** | S5 | A22 | 6-(3-Fluorphenyl)-N-(4-phenylbutan-2-yl)-5,6,7,8-tetrahydroimidazo[1,2-a]pyridin-2-carboxamid | 392,2 |
| **52** | S9 | A22 | 6-(4-Ethylphenyl)-N-(4-phenylbutan-2-yl)-5,6,7,8-tetrahydroimidazo[1,2-a]pyridin-2-carboxamid | 402,2 |

Geräte:

HPLC-System Waters 1525 Hochdruck binäre HPLC-Pumpe mit integriertem Splitter, MUX-UV 2488 Detektor (Waters Corp.) und inhouse entwickeltem Autosampler auf der Grundlage eines Cavro RSP 9452 (Cavro Scientific Inst., Inc.) Roboter
Workstation
ZQ2000 MS instrument (Waters Corp.) mit ESI-Interface integriert mit MUX (Waters Corp.)
Methode:
♦ **HPLC column:**
LiChroCART 30-4 Purospher STAR RP-18, endcapped, 3μm (Merck)
♦ **Gradient:**
Eluent (A): Acetonitril-$H_2O$=5:95 mit 20 mM $HCOONH_4$ /$NH_4OH$ Puffer, pH 7.4
Eluent (B): Acetonitril-$H_2O$=80:20 with 20 mM $HCOONH_4$ /$NH_4OH$ buffer, pH 7.4
Gradienten Programm:

| Min. | A% | B% |
|---|---|---|
| 0,0 | 95 | 5 |
| 2,5 | 5 | 95 |
| 4,3 | 5 | 95 |
| 4,4 | 95 | 5 |
| 5,0 | 95 | 5 |

♦ **Säulentemperatur:**
♦ **Flußrate:**
♦ **Proben-Konzentration:**
♦ **Proben Lösungsmittel:**
♦ **Injektionsvolumen:** 2.5 μl
♦ **Detektion:** UV 220 nm

**Festsubstanzen**

(6-Phenyl-5,6,7,8-tetrahydroimidazo[1,2-a]pyridin-2-yl)(4-(3-(trifluormethyl)phenyl)piperazin-1-yl)methanon 53

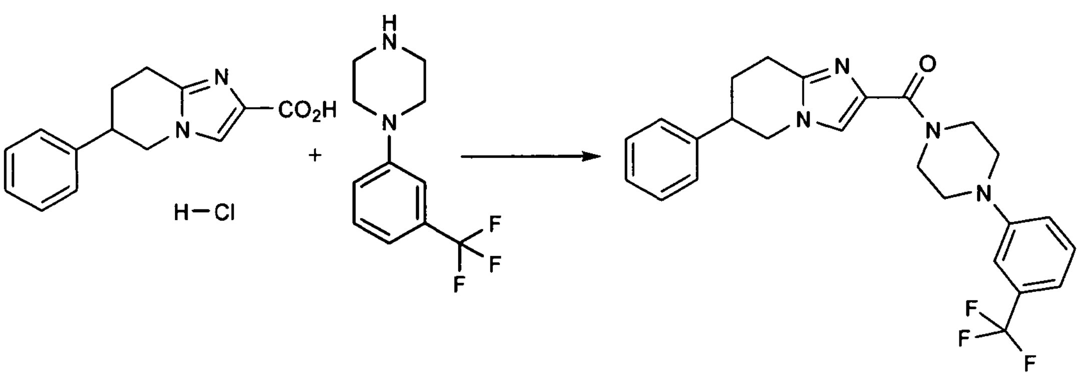

Zu einer Lösung des 6-Phenyl-5,6,7,8-tetrahydro-imidazo[1,2-a]pyridin-2-carboxylsäure Hydrochlorids (200 mg; 0,72 mmol) in DMF (1 ml) wurden TBTU (276 mg; 0,86 mmol) und N-Methylmorpholin (237 μl; 218 mg; 2,15 mmol) gegeben. Die Reationsmischung wurde 30 min. bei Raumtemperatur stark gerührt und dann mit 1-(3-Trifluormethyl-phenyl)piperazin (162 μl; 198 mg; 0,86 mmol) versetzt. Die Reaktionsmischung wurde 4 h bei Raumtemperatur gerührt, anschließend auf Wasser gegossen und dann mit Chloroform extrahiert. Die organische Phase wurde über $MgSO_4$ getrocknet, filtriert und eingeengt. Das Rohprodukt wurde säulenchromatographisch an Kieselgel (Chloroform) aufgereinigt.
Ausbeute: 191 mg (59%)
$^1$H NMR (600 MHz, DMSO-$d_6$) d ppm 2,02 - 2,14 (m, 2 H) 2,21 (s, 6 H) 2,46 - 2,55 (m, 3 H) 2,79 - 2,97 (m, 2 H) 3,06 - 3,19 (m, 1 H) 3,23 - 3,35 (m, 5 H) 3,64 (s, 3 H) 3,92 (t, $J$=11,71 Hz, 1 H) 4,22 (dd, $J$=12,46, 4,91 Hz, 1 H) 7,01 (s, 2 H) 7,09 (d, $J$=6,80 Hz, 1 H) 7,21 (br. s., 1 H) 7,25 (d, $J$=8,31 Hz, 1 H) 7,43 (t, $J$=7,93 Hz, 1 H) 7,56 (br. s., 1 H); MS: m/z 455,2 $[M+H]^+$

(6-(4-Methoxy-3,5-dimethylphenyl)-5,6,7,8-tetrahydroimidazo[1,2-a]pyridin-2-yl)(4-(3-(trifluormethyl)phenyl)piperazin-1-yl)methanon **54**

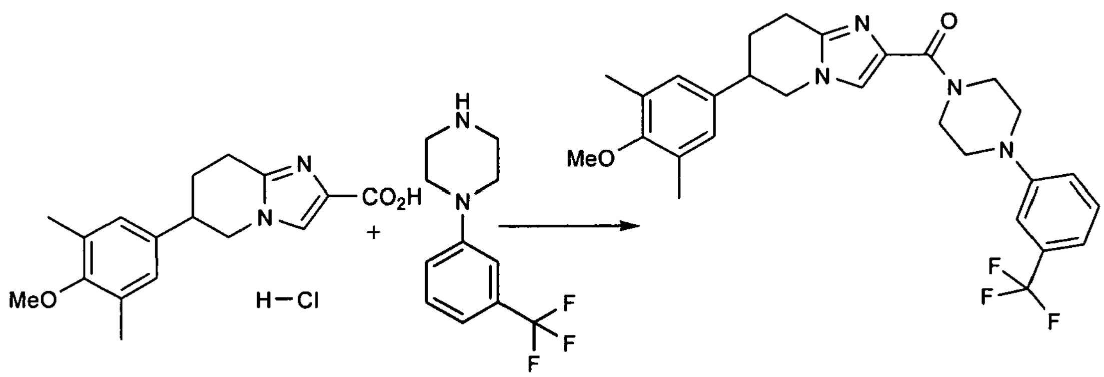

Zu einer Lösung des 6-(4-Methoxy-3,5-dimethylphenyl)-5,6,7,8-tetrahydroimidazo[1,2-a]pyridin-2-carboxylsäure Hydrochlorids (210 mg; 0,63 mmol) in DMF (1 ml) wurden TBTU (240 mg; 0,75 mmol) und N-Methylmorpholin (206 µl; 189 mg; 1,87 mmol) gegeben. Die Reationsmischung wurde 30 min. bei Raumtemperatur stark gerührt und dann mit 1-(3-Trifluormethylphenyl)piperazin (140 µl; 172 mg; 0,75 mmol) versetzt. Die Reaktionsmischung wurde 4 h bei Raumtemperatur gerührt, anschließend auf Wasser gegossen und dann mit Chloroform extrahiert. Die organische Phase wurde über $MgSO_4$ getrocknet, filtriert und eingeengt. Das Rohprodukt wurde säulenchromatographisch an Kieselgel (Chloroform) aufgereinigt. Ausbeute: 198 mg (62%)
$^1$H NMR (600 MHz, DMSO-$d_6$) d ppm 2,00 - 2,14 (m, 2 H) 2,21 (s, 6 H) 2,78 - 2,97 (m, 2 H) 3,08 - 3,20 (m, 1 H) 3,24 - 3,30 (m, 8 H) 3,64 (s, 3 H) 3,92 (t, $J$=11,71 Hz, 1 H) 4,22 (dd, $J$=12,46, 4,91 Hz, 1 H) 7,01 (s, 2 H) 7,09 (d, $J$=6,80 Hz, 1 H) 7,21 (br. s., 1 H) 7,25 (d, $J$=8,31 Hz, 1 H) 7,43 (t, $J$=7,93 Hz, 1 H) 7,56 (s, 1 H); MS: m/z 513.2 $[M+H]^+$

(4-Benzylpiperidin-1-yl)(6-(4-methoxy-3,5-dimethylphenyl)-5,6,7,8-tetrahydroimidazo[1,2-a]pyridin-2-yl)methanon **55**

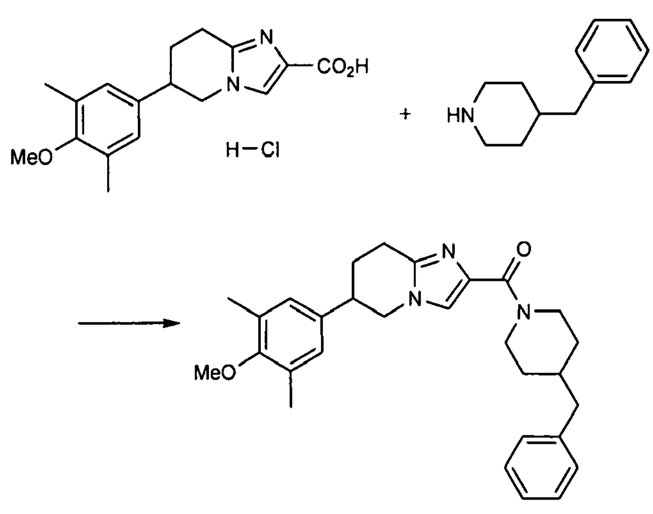

Zu einer Lösung des 6-(4-Methoxy-3,5-dimethylphenyl)-5,6,7,8-tetrahydroimidazo[1,2-a]pyridin-2-carboxylsäure Hydrochlorids (165 mg; 0,49 mmol) in DMF (1 ml) wurden TBTU (212 mg; 0,66 mmol) und N-Methylmorpholin (181 μl; 167 mg; 1,65 mmol) gegeben. Die Reationsmischung wurde 30 min. bei Raumtemperatur stark gerührt und dann mit 4-Benzylpiperidin (116 μl; 116 mg; 0,7 mmol) versetzt. Die Reaktionsmischung wurde 4 h bei Raumtemperatur gerührt und anschließend auf Wasser gegossen. Der ausgefallene Feststoff wurde filtriert.
Ausbeute: 182 mg (81 %)
$^1$H NMR (600 MHz, DMSO-$d_6$) d ppm 0,97 - 1,28 (m, 2 H) 1,51 - 1,69 (m, 2 H) 1,72 - 1,87 (m, 1 H) 1,97 - 2,11 (m, 2 H) 2,21 (s, 6 H) 2,50 - 2,56 (m, 4 H) 2,71 - 2,99 (m, 4 H) 3,01 - 3,21 (m, 1 H) 3,63 (s, 3 H) 3,77 - 3,99 (m, 1 H) 4,10 - 4,26 (m, 1 H) 7,00 (s, 2 H) 7,09 - 7,22 (m, 3 H) 7,28 (t, *J*=7,18 Hz, 2 H) 7,45 (br. s., 1 H); MS: m/z 458,3 $[M+H]^+$

(6-(4-Methoxy-3,5-dimethylphenyl)-5,6,7,8-tetrahydroimidazo[1,2-a]pyridin-2-yl)(1,3,3-trimethyl-6-azabicyclo[3.2.1]octan-6-yl)methanon **56**

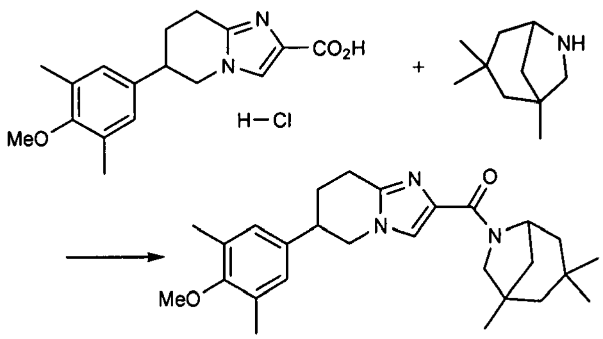

Zu einer Lösung des 6-(4-Methoxy-3,5-dimethylphenyl)-5,6,7,8-tetrahydroimidazo[1,2-a]pyridin-2-carboxylsäure Hydrochlorids (165 mg; 0,49 mmol) in DMF (1 ml) wurden TBTU (212 mg; 0,66 mmol) und N-Methylmorpholin (181 μl; 167 mg; 1,65 mmol) gegeben. Die Reationsmischung wurde 30 min. bei Raumtemperatur stark gerührt und dann mit 1,3,3-Trimethyl-6-azabicyclo[3.2.1.]octan (112 μl; 101 mg; 0,7 mmol) versetzt. Die Reaktionsmischung wurde 4 h bei Raumtemperatur gerührt und anschließend auf Wasser gegossen. Der ausgefallene Feststoff wurde filtriert.
Ausbeute: 180 mg (84%) Diastereomerengemisch
ESI-MS: m/z 436,3 $[M+H]^+$

(4-Benzylpiperidin-1-yl)(6-(4-fluor-3-methylphenyl)-5,6,7,8-tetrahydroimidazo[1,2-a]pyridin-2-yl)methanon **57**

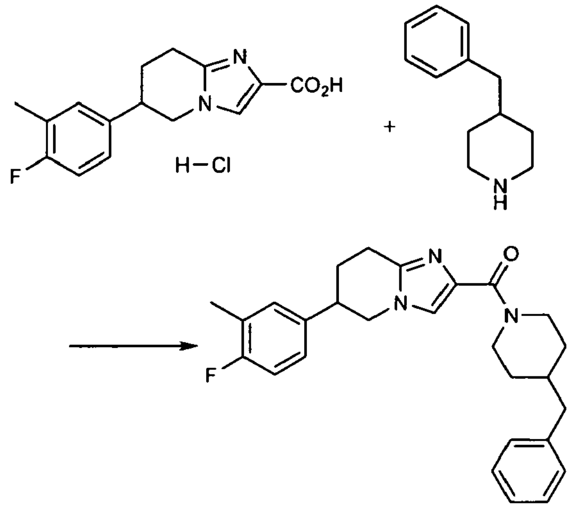

Zu einer Lösung des 6-(4-Fluor-3-methyl-phenyl)-5,6,7,8-tetrahydro-imidazo[1,2-a]pyridin-2-carboxylsäure Hydrochlorids (210 mg; 0,68 mmol) in DMF (1 ml) wurden TBTU (295mg; 0,92 mmol) und N-Methylmorpholin (253 µl; 232 mg; 2,3 mmol) gegeben. Die Reationsmischung wurde 30 min. bei Raumtemperatur stark gerührt und dann mit 4-Benzylpiperidin (162 µl; 161 mg; 0,9 mmol) versetzt. Die Reaktionsmischung wurde 4 h bei Raumtemperatur gerührt und anschließend auf Wasser gegossen. Der ausgefallene Feststoff wurde filtriert und mit Petrolether gewaschen.
Ausbeute: 205 mg (72%)
$^1$H NMR (600 MHz, DMSO-$d_6$) d ppm 1,02 - 1,18 (m, 2 H) 1,54 - 1,66 (m, 2 H) 1,73 - 1,85 (m, 1 H) 2,02 - 2,14 (m, 2 H) 2,23 (s, 3 H) 2,51 - 2,56 (m, 2 H) 2,76 - 2,97 (m, 4 H) 3,16 - 3,26 (m, 1 H) 3,25 - 3,30 (m, 2 H) 3,92 (t, *J*=11,71 Hz, 1 H) 4,21 (dd, *J*=12,46, 4,91 Hz, 1 H) 7,10 (t, *J*=9,06 Hz, 1 H) 7,14 - 7,23 (m, 4 H) 7,28 (t, *J*=6,80 Hz, 3 H) 7,46 (s, 1 H) ); MS: m/z 432,2 $[M+H]^+$

(6-(4-Fluor-3-methylphenyl)-5,6,7,8-tetrahydroimidazo[1,2-a]pyridin-2-yl)(4-(3-(trifluormethyl)phenyl)piperazin-1-yl) methanon **58**

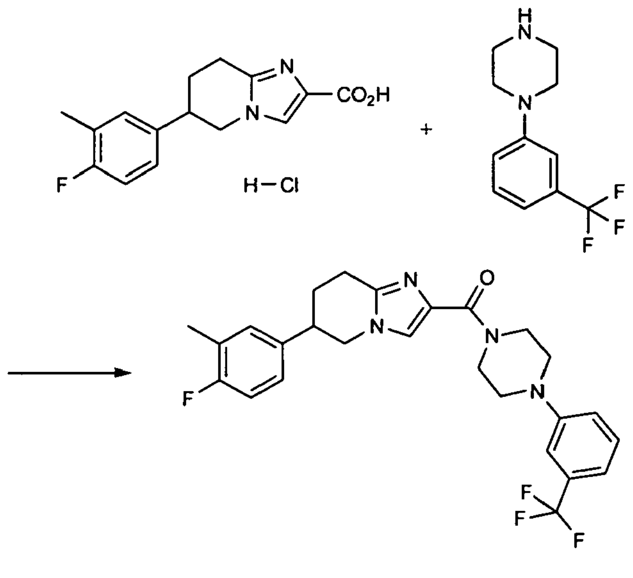

Zu einer Lösung des 6-(4-Fluor-3-methyl-phenyl)-5,6,7,8-tetrahydro-imidazo[1,2-a]pyridin-2-carboxylsäure Hydrochlorids (210 mg; 0,68 mmol) in DMF (1 ml) wurden TBTU (295 mg; 0,92 mmol) und N-Methylmorpholin (253 µl; 232 mg; 2,3 mmol) gegeben. Die Reationsmischung wurde 30 min. bei Raumtemperatur stark gerührt und dann mit

1-(3-Trifluormethylphenyl)piperazin (173 µl; 212 mg; 0,92 mmol) versetzt. Die Reaktionsmischung wurde 4 h bei Raumtemperatur gerührt und auf Wasser gegossen. Der ausgefallene Feststoff wurde säulenchromatographisch an Kieselgel (Chloroform) aufgereinigt.
Ausbeute: 166 mg (50%)
$^1$H NMR (600 MHz, DMSO-$d_6$) d ppm 2,00 - 2,19 (m, 2 H) 2,23 (s, 3 H) 2,78 - 3,02 (m, 2 H) 3,11 - 3,29 (m, 5 H) 3,55 - 3,89 (m, 2 H) 3,95 (t, *J*=11,71 Hz, 1 H) 4,25 (dd, *J*=12,46, 4,91 Hz, 1 H) 4,30 - 4,66 (m, 2 H) 7,01 - 7,16 (m, 2 H) 7,17 - 7,24 (m, 2 H) 7,25 (d, *J*=9,06 Hz, 1 H) 7,29 (d, *J*=7,55 Hz, 1 H) 7,43 (t, *J*=7,93 Hz, 1 H) 7,57 (s, 1 H) ; MS: m/z 487,2 $[M+H]^+$

(4-Benzhydrylpiperazin-1-yl)(6-(4-methoxy-3,5-dimethylphenyl)-5,6,7,8-tetrahydroimidazo[1,2-a]pyridin-2-yl)methanon **59**

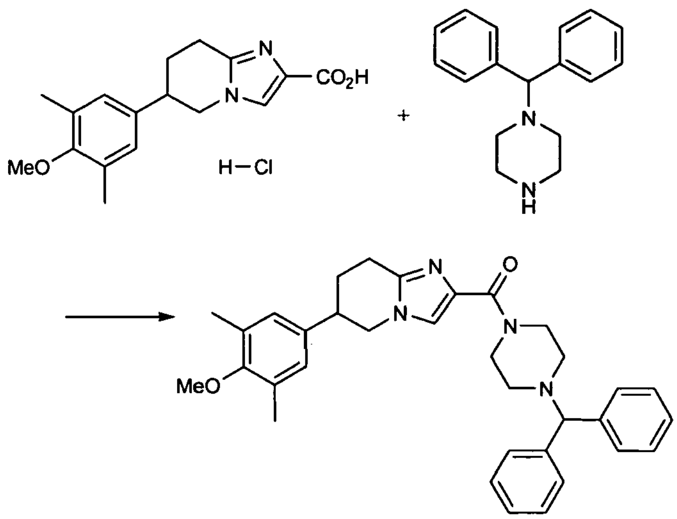

Zu einer Lösung des 6-(4-Methoxy-3,5-dimethylphenyl)-5,6,7,8-tetrahydroimidazo[1,2-a]pyridin-2-carboxylsäure Hydrochlorids (165 mg; 0,49 mmol) in DMF (1 ml) wurden TBTU (212 mg; 0,66 mmol) und N-Methylmorpholin (181 µl; 167 mg; 1,65 mmol) gegeben. Die Reationsmischung wurde 30 min. bei Raumtemperatur stark gerührt und dann mit Diphenylmethylpiperazin (166 mg; 0,7 mmol) versetzt. Die Reaktionsmischung wurde 4 h bei Raumtemperatur gerührt, auf Wasser gegossen. Der ausgefallene Feststoff wurde filtriert.
Ausbeute: 232 mg (88%)
$^1$H NMR (600 MHz, DMSO-$d_6$) d ppm 1,96 - 2,12 (m, 2 H) 2,20 (s, 6 H) 2,26 - 2,38 (m, 4 H) 2,45 - 2,54 (m, 2 H) 2,72 - 2,94 (m, 2 H) 3,01 - 3,18 (m, 1 H) 3,55 - 3,72 (m, 4 H) 3,87 (t, *J*=11,71 Hz, 1 H) 4,18 (dd, *J*=12,09, 4,53 Hz, 2 H) 4,35 (s, 1 H) 6,99 (s, 2 H) 7,20 (t, *J*=7,18 Hz, 2 H) 7,30 (t, *J*=7,55 Hz, 4 H) 7,43 (d, *J*=7,55 Hz, 4 H) 7,47 (s, 1 H) ; MS: m/z 535,3 $[M+H]^+$

6-(3-Methoxyphenyl)-N-(4-phenylbutan-2-yl)-5,6,7,8-tetrahydroimidazo[1,2-a]pyridin-2-carboxamid **60**

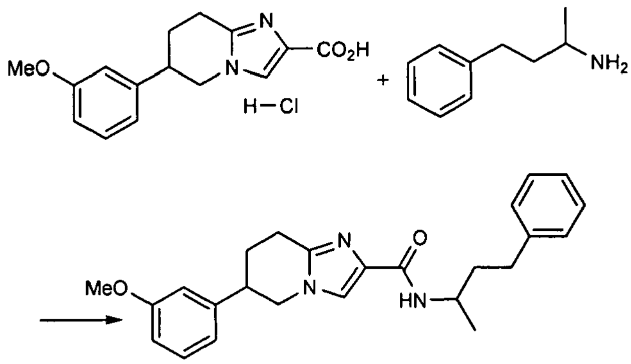

Zu einer Lösung des 6-(3-Methoxy-phenyl)-5,6,7,8-tetrahydro-imidazo[1,2-a]pyridin-2-carboxylsäure Hydrochlorids (250 mg; 0,8 mmol) in DMF (1 ml) wurden TBTU (310 mg; 0,97 mmol) und N-Methylmorpholin (267 μl; 245 mg; 2,4 mmol) gegeben. Die Reationsmischung wurde 30 min. bei Raumtemperatur stark gerührt und dann mit 1-Methyl-3-phenylpropylamin (157 μl; 145 mg; 1,0 mmol) versetzt. Die Reaktionsmischung wurde 4 h bei Raumtemperatur gerührt, auf Wasser gegossen und dann mit Chloroform extrahiert. Die organische Phase wurde über $MgSO_4$ getrocknet, filtriert und eingeengt. Das Rohprodukt wurde säulenchromatographisch an Kieselgel (Chloroform) aufgereinigt.
Ausbeute: 183 mg (56%)
$^1$H NMR (600 MHz, DMSO-$d_6$) d ppm 1,14 (d, *J*=6,04 Hz, 3 H) 1,63 - 1,77 (m, 1 H) 1,81 - 1,91 (m, 1 H) 2,06 - 2,19 (m, 2 H) 2,52 - 2,63 (m, 2 H) 2,80 - 2,96 (m, 2 H) 3,18 - 3,27 (m, 1 H) 3,75 (s, 3 H) 3,93 - 4,05 (m, 2 H) 4,25 (dd, *J*=12,09, 4,53 Hz, 1 H) 6,84 (d, *J*=8,31 Hz, 1 H) 6,90 - 6,98 (m, 2 H) 7,12 - 7,22 (m, 3 H) 7,23 - 7,31 (m, 3 H) 7,50 (s, 1 H) 7,58 (d, *J*=8,31 Hz, 1 H) ; MS: m/z 404,2 $[M+H]^+$

**Synthese der Verbindungen 61 - 82**

Zu einer Lösung der Carbonsäure (500 mg) in DMF (2,5 ml) wurden TBTU (1,5 Äq.) und N-Methylmorpholin (3,5 Äq.) gegeben. Die Reationsmischung wurde 30 min. bei Raumtemperatur stark gerührt und dann mit dem entsprechenden Amin (1,1 Äq.) versetzt. Die Reaktionsmischung wurde über Nacht bei Raumtemperatur gerührt. Nach beendeter Reaktion wurde mit Ethylacetat verdünnt und nacheinander mit ges. Ammoniumchlorid-Lösung, ges. Natriumchlorid-Lösung, Natriumhydrogencarbonat-Lösung und ges. Natriumchlorid-Lösung gewaschen. Die organische Phase wurde über Natriumsulfat getrocknet und eingeengt. Das Rohprodukt wurde säulenchromatographisch mit Hilfe des in der nachfolgenden Tabelle angegebenen Laufmittels an Kieselgel aufgereinigt.

| Nr. | Struktur | Ausbeute % | Laufmittel | MS m/z $[M+H]^+$ |
|---|---|---|---|---|
| **61** | 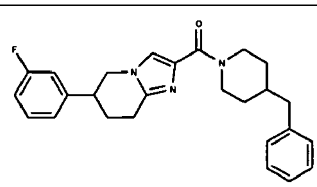 (4-Benzylpiperidin-1-yl)(6-(3-fluorphenyl)-5,6,7,8-tetrahydroimidazo[1,2-a]pyridin-2-yl) methanon | 80 | 80 % Ethylacetat in Hexan | 418,2 |
| **62** | 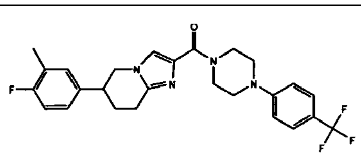 (6-(4-Fluor-3-methylphenyl)-5,6,7,8-tetrahydroimidazo[1,2-a]pyridin-2-yl) (4-(4-(trifluormethyl)phenyl)piperazin-1-yl) methanon | 28 | 60 % Ethylacetat in Hexan | 487,2 |

(fortgesetzt)

| Nr. | Struktur | Ausbeute % | Laufmittel | MS m/z [M+H]+ |
|---|---|---|---|---|
| **63** | 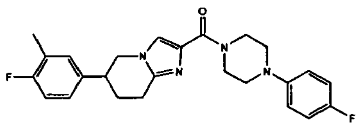 (6-(4-Fluor-3-methylphenyl)-5,6,7,8-tetrahydroimidazo[1,2-a]pyridin-2-yl)(4-(4-fluorphenyl)piperazin-1-yl)methanon | 32 | 5 % Methanol in DCM | 437,2 |
| **64** | 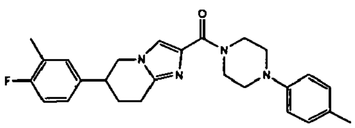 (6-(4-Fluor-3-methylphenyl)-5,6,7,8-tetrahydroimidazo[1,2-a]pyridin-2-yl)(4-(4-methylphenyl)piperazin-1-yl)methanon | 68 | 70 % Ethylacetat in Hexan | 433,2 |
| **65** | 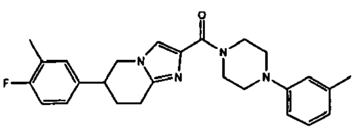 (6-(4-Fluor-3-methylphenyl)-5,6,7,8-tetrahydroimidazo[1,2-a]pyridin-2-yl)(4-(3-methylphenyl)piperazin-1-yl)methanon | 70 | 70 % Ethylacetat in Hexan | 433,2 |
| **66** | 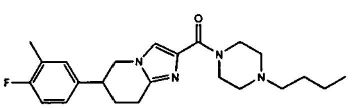 (4-Butylpiperazin-1-yl)(6-(4-fluor-3-methylphenyl)-5,6,7,8-tetrahydroimidazo[1,2-a]pyridin-2-yl)methanon | 56 | 80 % Ethylacetat in Hexan | 399,2 |
| **67** | 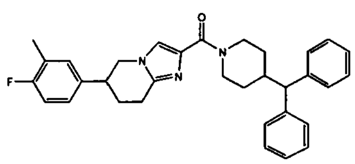 (4-Benzhydrylpiperidin-1-yl)(6-(4-fluor-3-methylphenyl)-5,6,7,8-tetrahydroimidazo[1,2-a]pyridin-2-yl)methanon | 67 | 80 % Ethylacetat in Hexan | 508,3 |
| **68** | 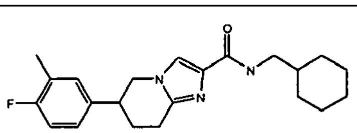 N-(Cyclohexylmethyl)-6-(4-fluor-3-methylphenyl)-5,6,7,8-tetrahydroimidazo[1,2-a]pyridin-2-carboxamid | 54 | 70 % Ethylacetat in Hexan | 370,2 |
| **69** | 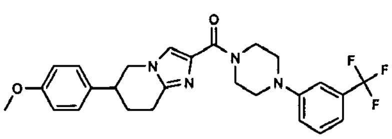 | 40 | 70 % Ethylacetat in Hexan | 485,2 |

(fortgesetzt)

| Nr. | Struktur | Ausbeute % | Laufmittel | MS m/z [M+H]+ |
|---|---|---|---|---|
| | (6-(4-Methoxyphenyl)-5,6,7,8-tetrahydroimidazo[1,2-a]pyridin-2-yl) (4-(3-(trifluormethyl)phenyl)piperazin-1-yl) methanon | | | |
| **70** | 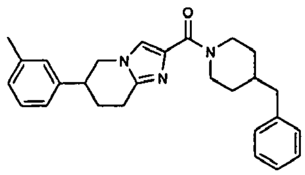 (4-Benzylpiperidin-1-yl)(6-m-tolyl-5,6,7,8-tetrahydroimidazo[1,2-a]pyridin-2-yl) methanon | 58 | 80 % Ethylacetat in Hexan | 414,2 |
| **71** | 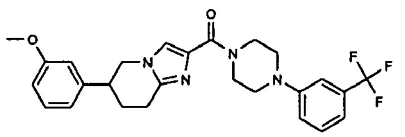 (6-(3-Methoxyphenyl)-5,6,7,8-tetrahydroimidazo[1,2-a]pyridin-2-yl) (4-(3-(trifluormethyl)phenyl)piperazin-1-yl) methanon | 76 | 90 % Ethylacetat in Hexan | 485,2 |
| **72** | 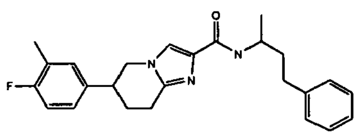 6-(4-Fluor-3-methylphenyl)-N-(4-phenylbutan-2-yl)-5,6,7,8-tetrahydroimidazo [1,2-a]pyridin-2-carboxamid | 66 | 80 % Ethylacetat in Hexan | 406,2 |
| **73** | 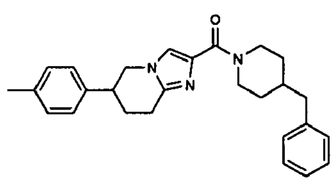 4-Benzylpiperidin-1-yl)(6-p-tolyl-5,6,7,8-tetrahydroimidazo[1,2-a]pyridin-2-yl) methanon | 22 | 80 % Ethylacetat in Hexan | 414,2 |
| **74** | 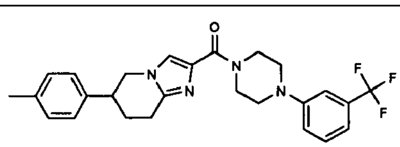 (6-p-Tolyl-5,6,7,8-tetrahydroimidazo[1,2-a] pyridin-2-yl)(4-(3-(trifluormethyl) phenyl) piperazin-1-yl)methanon | 19 | 80 % Ethylacetat in Hexan | 469,2 |
| **75** | 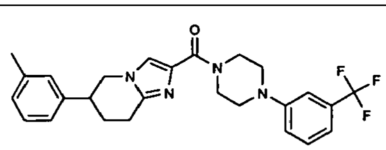 | 33 | 80 % Ethylacetat in Hexan | 469,2 |

(fortgesetzt)

| Nr. | Struktur | Ausbeute % | Laufmittel | MS m/z [M+H]+ |
|---|---|---|---|---|
| | (6-m-Tolyl-5,6,7,8-tetrahydroimidazo[1,2-a] pyridin-2-yl)(4-(3-(trifluormethyl) phenyl) piperazin-1-yl)methanon | | | |
| **76** | 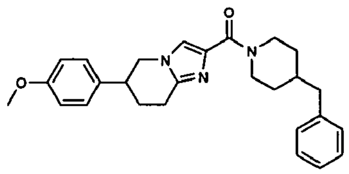 (4-Benzylpiperidin-1-yl)(6-(4-methoxyphenyl)-5,6,7,8-tetrahydroimidazo [1,2-a]pyridin-2-yl)methanon | 35 | 75 % Ethylacetat in Hexan | 430,2 |
| **77** | 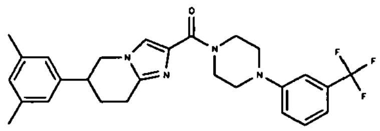 (6-(3,5-Dimethylphenyl)-5,6,7,8-tetrahydroimidazo[1,2-a]pyridin-2-yl) (4-(3-(trifluormethyl)phenyl)piperazin-1-yl) methanon | 50 | 80 % Ethylacetat in Hexan | 483,2 |
| **78** | 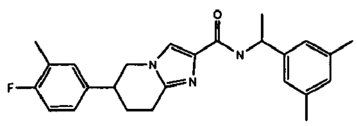 N-(1-(3,5-Dimethylphenyl)ethyl)-6-(4-fluor-3-methylphenyl)-5,6,7,8-tetrahydroimidazo[1,2-a]pyridine-2-carboxamid | 41 | 75 % Ethylacetat in Hexan | 406,2 |
| **79** | 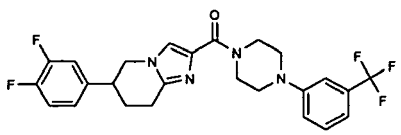 (6-(3,4-Difluorphenyl)-5,6,7,8-tetrahydroimidazo[1,2-a]pyridin-2-yl) (4-(3-(trifluormethyl)phenyl)piperazin-1-yl) methanon | | | 491,2 |
| **80** | 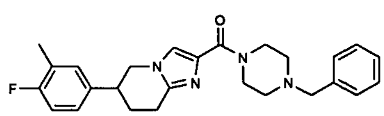 (4-Benzylpiperazin-1-yl)(6-(4-fluor-3-methylphenyl)-5,6,7,8-tetrahydroimidazo[1,2-a]pyridin-2-yl)methanon | | | 433,2 |
| **81** | 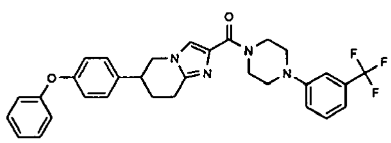 (6-(4-Phenoxyphenyl)-5,6,7,8-tetrahydroimidazo[1,2-a]pyridin-2-yl) (4-(3-(trifluormethyl)phenyl)piperazin-1-yl) methanon | | | 547,2 |

(fortgesetzt)

| Nr. | Struktur | Ausbeute % | Laufmittel | MS m/z $[M+H]^+$ |
|---|---|---|---|---|
| **82** | 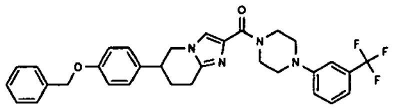 (6-(4-(Benzyloxy)phenyl)-5,6,7,8-tetrahydroimidazo[1,2-a]pyridin-2-yl) (4-(3-(trifluormethyl)phenyl)piperazin-1-yl) methanon | | | 561,2 |

**Biologische Daten**

**Fluoreszenzassay unter Verwendung eines 'voltage sensitive dyes'**

Humane, KCNQ2/3-Kanäle exprimierende CHO-K1-Zellen werden in Zellkulturflaschen (z.B. 80 $cm^2$ TC flasks, Nunc) mit MEM Alpha Medium (1x, flüssig, Invitrogen, #22571), 10 % Fetal Calf Serum (FCS) (Invitrogen, #10270-106, hitzeinaktiviert) und den notwendigen Selektionsantibiotika bei 37˚C, 5 % $CO_2$ und 95 % Luftfeuchtigkeit adhärent kultiviert.

Vor Aussaat für die Messungen werden die Zellen mit einem 1 x DPBS-Puffer ohne $Ca^{2+}/Mg^{2+}$ (z.B. Invitrogen, #14190-094) gewaschen und mittels Accutase (PAA Laboratories, #L11-007) vom Boden des Kulturgefäßes abgelöst (Inkubation mit Accutase für 15 min bei 37˚C). Die Bestimmung der dann vorliegenden Zellzahl wird mit einem CASY™ cell counter (Modell TCC, Schärfe System) durchgeführt, um anschließend 20.000 Zellen/well/100 µl des beschriebenen Nährmediums auf die 96 well-Messplatten des Typs Corning™ CellBIND™ (Flat Clear Bottom Black Polystyrene Microplates, #3340) auszubringen. Danach erfolgt eine einstündige Inkubation bei Raumtemperatur ohne Begasung oder Regelung der Luftfeuchtigkeit, gefolgt von einer 24stündigen Inkubation bei 37˚C, 5 % $CO_2$ und 95 % Luftfeuchtigkeit.
Der spannungssensitive Fluoreszenzfarbstoff aus dem Membrane Potential Assay Kit (Red™ Bulk format part R8123 for FLIPR, Molecular Devices™) wird vorbereitet, indem der Inhalt eines Gefäßes *Membrane Potential Assay Kit Red Component A* in 200 ml Extrazellulären Puffers (ES-Puffer, 120 mM NaCl, 1 mM KCl, 10 mM HEPES, 2 mM $CaCl_2$, 2 mM $MgCl_2$, 10 mM Glucose; pH 7,4) gelöst wird. Nach Abnahme des Nährmediums werden die Zellen mit 200 µl ES-Puffer gewaschen, anschließend mit 100 µl der oben angesetzten Farbstofflösung überschichtet und 45 min bei Raumtemperatur unter Lichtverschluss inkubiert.
Die Fluoreszenzmessungen werden mit einem BMG Labtech FLUOstar™- oder BMG Labtech POLARstar™-Instrument durchgeführt (525 nm Exication, 560 nm Emission, Bottom Read mode). Nach der Farbstoff-Inkubation werden 50 µl der zu testenden Substanzen in den gewünschten Konzentrationen oder 50 µl ES-Puffer zwecks Kontrolle in separate Cavitäten der Messplatte gegeben und 30 min bei Raumtemperatur unter Abschirmung von Licht inkubiert. Anschließend misst man für 5 min die Fluoreszenzintensität des Farbstoffs und ermittelt so zu einem festgelegten und gleichbleibenden Zeitpunkt den Fluoreszenzwert $F_1$ eines jeden wells. Daraufhin erfolgt Zugabe von 15 µl einer 100 mM KCl-Lösung (Endkonzentration 92 mM) in jedes well. Die Veränderung der Fluoreszenz wird anschließend so lange gemessen, bis alle relevanten Messwerte erhalten sind (vornehmlich 5-30 min). Zu einem festgelegten Zeitpunkt nach KCl-Applikation wird ein Fluoreszenzwert $F_2$ ermittelt, in diesem Falle zum Zeitpunkt des Fluoreszenzpeaks.

Zur Berechnung wird die Fluoreszenzintensität $F_2$ mit der Fluoreszenzintensität $F_1$ verglichen und daraus die agonistische Aktivität der Zielverbindung auf den Kaliumkanal ermittelt. $F_2$ und $F_1$ werden hierfür wie folgt verrechnet:

$$\left(\frac{F_2 - F_1}{F_1}\right) \times 100 = \frac{\Delta F}{F} (\%)$$

Um zu ermitteln, ob eine Substanz eine agonistische Aktivität besitzt, kann z.B. $\frac{\Delta F}{F}$ mit $\left(\frac{\Delta F}{F}\right)_K$ von Kontrollzellen

verglichen werden. $\left(\frac{\Delta F}{F}\right)_K$ wird ermittelt, indem man dem Reaktionsansatz anstatt der zu testenden Substanz lediglich die Pufferlösung zusetzt, den Wert $F_{1K}$ der Fluoreszenzintensität bestimmt, die Kaliumionen wie oben beschrieben zugibt und einen Wert $F_{2K}$ der Fluoreszenzintensität misst. Dann werden $F_{2K}$ und $F_{1K}$ wie folgt verrechnet:

$$\left(\frac{F_{2K} - F_{1K}}{F_{1K}}\right) \times 100 = \left(\frac{\Delta F}{F}\right)_K (\%)$$

Eine Substanz besitzt eine agonistische Aktivität auf den Kaliumkanal, wenn $\frac{\Delta F}{F}$ größer als $\left(\frac{\Delta F}{F}\right)_K$ ist:

$$\frac{\Delta F}{F} \rangle \left(\frac{\Delta F}{F}\right)_K$$

Unabhängig vom Vergleich von $\frac{\Delta F}{F}$ mit $\left(\frac{\Delta F}{F}\right)_K$ lässt sich auch auf eine agonistische Aktivität einer Zielverbindung schließen, wenn mit steigender Dosierung der Zielverbindung eine Zunahme von $\frac{\Delta F}{F}$ zu beobachten ist.

Kalkulationen von $EC_{50}$- und $IC_{50}$-Werten werden mit Hilfe der Software 'Prism 4' (GraphPad Software™) durchgeführt.

| Nr. | | % Stimulation [10 μmol] Mittelwert | $EC_{50}$[μM] |
|---|---|---|---|
| **1** | | 16,2 | |
| **2** | | 20,0 | |
| **3** | | 16,7 | |
| **4** | | 20,7 | |
| **5** | | 15,5 | |
| **6** | | | 7,4 |
| **7** | | 30,0 | |
| **8** | | 29,1 | |
| **9** | | | 11,92 |
| **10** | | 15,7 | |
| **11** | | 38,5 | |
| **12** | | 51,8 | |
| **13** | | 18,0 | |
| **14** | | 24,7 | |
| **15** | | 16,8 | |
| **16** | | | 3,95 |
| **17** | | 18,5 | |
| **18** | | 18,6 | |

(fortgesetzt)

| Nr. | | % Stimulation [10 μmol] Mittelwert | $EC_{50}$[μM] |
|---|---|---|---|
| **19** | | | 6,21 |
| **20** | | | 11,61 |
| **21** | | | 8,4 |
| **22** | | 18,0 | |
| **23** | | | 5,81 |
| **24** | | | 6,14 |
| **25** | | | 2,33 |
| **26** | | 17,4 | |
| **27** | | | 6,35 |
| **28** | | | 9,38 |
| **29** | | 26,2 | |
| **30** | | 20,9 | |
| **31** | | 17,6 | |
| **32** | | 21,2 | |
| **33** | | 24,5 | |
| **34** | | 32,2 | |
| **35** | | 20,9 | |
| **36** | | 21,7 | |
| **37** | | 15,5 | |
| **38** | | 25,9 | |
| **39** | | 17,8 | |
| **40** | | 16,6 | |
| **41** | | 29,4 | |
| **42** | | 18,1 | |
| **43** | | 16,3 | |
| **44** | | 27,5 | |
| **45** | | | 7,98 |
| **46** | | | 3,98 |
| **47** | | 63,5 | |
| **48** | | 33,0 | |
| **49** | | 15,1 | |
| **50** | | 25,7 | |
| **51** | | 16,2 | |
| **52** | | 34,2 | |
| **53** | | | 8,34 |
| **54** | | | 2,45 |
| **55** | | | 5,42 |
| **56** | | | 11,31 |

(fortgesetzt)

| Nr. | | % Stimulation [10 μmol] Mittelwert | $EC_{50}$[μM] |
|---|---|---|---|
| **57** | | | 9,71 |
| **58** | | | 4,46 |
| **59** | | | 8,4 |
| **60** | | | 6,4 |

## Abkürzungsverzeichnis

| | |
|---|---|
| CDI | 1,1'-Carbonyldiimidazol |
| d | Tag(e) |
| dba | Dibenzylidenaceton |
| DCE | 1,2-Dichlorethan |
| DCC | N,N'-Dicyclohexylcarbodiimid |
| DCM | Dichlormethan |
| DIEA | Diisopropylethylamin |
| DMA | N,N-Dimethylacetamid |
| DMAP | 4-(Dimethylamino)-pyridin |
| DMF | Dimethylformamid |
| dppf | 1,1'-Bis(diphenylphosphinoferrocen) |
| EDCI | N-(3-Dimethylaminopropyl)-N'-ethyl-carbodiimid |
| EDTA | Ethylendiamin-N,N,N',N'-tetraessigsäure |
| h | Stunde(n) |
| HBTU | O-(Benzotriazol-1-yl)-N,N,N',N'-tetramethyluroniumhexafluorphosphat |
| HOBt | 1-Hydroxy-1H-benzotriazol |
| M | molar |
| min. | Minute(n) |
| N | Normal |
| OAc | Acetat |
| $P(Cy)_3$ | Tricyclohexylphosphin |
| $P(o\text{-}Tolyl)_3$ | Tri-o-Tolylphosphin |
| $PPh_3$ | Triphenylphosphin |
| quant. | quantitativ |
| TBTU | *O*-(Benzotriazol-1-yl)-*N,N,N'N'*-tetramethyluronium-tetrafluorborat |
| TEA | Triethylamin |
| TFA | Trifluoressigsäure |
| THF | Tetrahydrofuran |

## Patentansprüche

**1.** Substituierte Tetrahydroimidazopyridin-Verbindungen der allgemeinen Formel I,

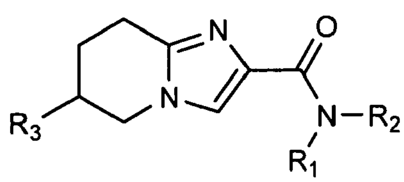

**I**

worin
$R^1$ und $R^2$ unabhängig voneinander für H; $C_{1-10}$-Alkyl, $C_{1-4}$-Alkyl-Aryl, $C_{1-4}$-Alkyl-Heteroaryl, $C_{1-4}$-Alkyl-$C_{3-8}$-Cycloalkyl, $C_{1-4}$-Alkyl-Heterocyclyl oder $C_{3-8}$-Cycloalkyl stehen,
wobei die Alkyl-, Aryl-, Heteroaryl-, Heterocyclyl- und Cycloalkylreste jeweils unsubstituiert oder einfach oder mehrfach substituiert sind und $R^1$ und $R^2$ nicht gleichzeitig H bedeuten;
oder die Reste $R^1$ und $R^2$ unter Einschluss des Stickstoffs einen vier bis achtgliedrigen, gegebenenfalls über eine $C_1$- oder $C_2$-Alkylkette verbrückten Heterocyclyl-Ring bilden, der ein weiteres Heteroatom enthalten und substituiert oder unsubstituiert sein kann,
und
$R^3$ Phenyl, einfach oder mehrfach substituiert oder unsubstituiert bedeutet,
mit der Maßgabe, dass wenn $R^1$ oder $R^2$ H, Alkyl oder Phenylalkyl bedeutet, der andere Rest $R^1$ oder $R^2$ nicht für eine Gruppe der Formel X1 steht

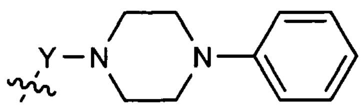

X1,

wobei der Phenylrest in der Formel X1 unsubstituiert oder substituiert sein kann und Y für eine $C_{2-5}$-Alkylkette oder $-(CH_2)_o$-Z-$(CH_2)_p$- steht, wobei Z für Cyclopentyl, Cyclohexyl oder Cycloheptyl steht und o und p 0, 1, 2 oder 3 ist, wobei die Summe aus o und p höchstens 3 beträgt,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

**2.** Substituierte Tetrahydroimidazopyridin-Verbindungen gemäß Anspruch 1,
worin
"Alkyl substituiert", "Heterocyclyl substituiert" und "Cycloalkyl substituiert" für die Substitution eines Wasserstoffrestes durch F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, C(=O)O$C_{1-6}$-Alkyl, Phenyl oder Benzyl steht,
und "Aryl substituiert", "Phenyl substituiert" und "Heteroaryl substituiert" für die ein-oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)-Aryl; C(=O)$C_{1-6}$-Alkyl, C(=O)NH$C_{1-6}$-Alkyl; C(=O)-N-Morpholin; C(=O)-Piperidin; (C=O)-Pyrrolidin; (C=O)-Piperazin; $NHSO_2C_{1-6}$-Alkyl,
NHCO$C_{1-6}$-Alkyl, $CO_2H$, $CH_2SO_2$-Phenyl, $CO_2$-$C_{1-6}$-Alkyl, $OCF_3$, $CF_3$,

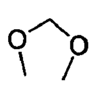 , 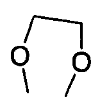 ,

$C_{1-6}$-Alkyl, Pyrolidinyl, Piperidinyl, Morpholinyl, Benzyloxy, Phenoxy, Phenyl, Pyridyl, Alkylaryl, Thienyl oder Furyl; steht,
wobei die Reste $C_{1-6}$-Alkyl, Pyrolidinyl, Piperidinyl, Morpholinyl, Benzyloxy, Phenoxy, Phenyl, Pyridyl, Alkylaryl, Thienyl oder Furyl können ihrerseits mit F, Cl, Methoxy, Ethoxy, $CF_3$, CN, $CH_3$, OH, $OCF_3$, $SCF_3$, oder $NO_2$ substituiert sein können.

**3.** Substituierte Tetrahydroimidazopyridin-Verbindungen gemäß einem der Ansprüche 1 oder 2,
worin
$R^1$ und $R^2$ unabhängig voneinander für $C_{1-10}$-Alkyl, $C_{1-4}$-Alkyl-Aryl, $C_{1-4}$-Alkyl-Heteroaryl, $C_{1-4}$-Alkyl-$C_{3-8}$-Cycloalkyl, oder $C_{3-8}$-Cycloalkyl stehen
wobei die Alkyl-, Aryl-, Heteroaryl- und Cycloalkylreste jeweils unsubstituiert oder einfach oder mehrfach substituiert sind.

**4.** Substituierte Tetrahydroimidazopyridin-Verbindungen gemäß einem der Ansprüche 1 oder 2, worin die Reste $R^1$ und $R^2$ unter Einschluss des Stickstoffs einen vier bis achtgliedrigen, gegebenenfalls über eine $C_1$- oder $C_2$-Alkylkette verbrückten Ring bilden, der ein weiteres Heteroatom aus der Gruppe O, N oder S enthalten und substituiert oder unsubstituiert sein kann.

**5.** Substituierte Tetrahydroimidazopyridin-Verbindungen gemäß Anspruch 3, worin $R^1$ und $R^2$ unabhängig voneinander für H; Benzyl, Phenethyl, Methylpyridyl, Cyclopropyl, n-Pentyl, n-Butyl, n-Hexyl, sec-Butyl, Propylethyl oder Methylcyclohexyl,
jeweils unsubstituiert oder einfach oder mehrfach substituiert mit Methoxy, F, $CH_3$, $CF_3$ oder

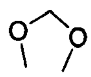;

stehen.

**6.** Substituierte Tetrahydroimidazopyridin-Verbindungen gemäß Anspruch 4, worin $R^1$ und $R^2$ einen fünf- bis siebengliedrigen Ring bilden, der ein weiteres Stickstoffatom enthalten und unsubsubstituiert oder einfach oder mehrfach substituiert mit $C(O)OC_2H_5$; $C(O)C_{1-6}$-Alkyl; Methyl, n-Butyl oder Acetyl; Phenyl oder Benzyl jeweils unsubstituiert oder substituiert mit Phenyl F, Cl, Br, I, CN, $CH_3$, $C_2H_5$, $NH_2$, $NO_2$, SH, $CF_3$, OH, $OCH_3$, $OCF_3$, $SCF_3$, $SCH_3$, $OC_2H_5$ oder $N(CH_3)_2$.

**7.** Substituierte Tetrahydroimidazopyridin-Verbindungen gemäß Anspruch 1, worin $R^3$ Phenyl, unsubstituiert oder einfach oder mehrfach substituiert mit F, Cl, Br, I, CN, $CH_3$, $C_2H_5$, $NH_2$, $NO_2$, SH, $CF_3$, OH, $OCH_3$, $OCF_3$, $SCF_3$, $SCH_3$, Phenoxy, $OC_2H_5$ oder $N(CH_3)_2$, bedeutet.

**8.** Tetrahydroimidazopyridin-Derivate gemäß Anspruch 1 aus der Gruppe

1 (4-Benzylpiperidin-1-yl)(6-phenyl-5,6,7,8-tetrahydroimidazo[1,2-a]pyridin -2-yl)methanon
2 N-Benzyl-N-phenethyl-6-phenyl-5,6,7,8-tetrahydroimidazo[1,2-a]pyridin-2-carboxamid
3 (6-Phenyl-5,6,7,8-tetrahydroimidazo[1,2-a]pyridin-2-yl)(1,3,3-trimethyl-6-azabicyclo[3.2.1]octan-6-yl)methanon
4 (6-(3-Fluorphenyl)-5,6,7,8-tetrahydroimidazo[1,2-a]pyridin-2-yl)(1,3,3-trimethyl-6-azabicyclo[3.2.1]octan-6-yl)methanon
5 N-Benzyl-6-(4-methoxy-3,5-dimethylphenyl)-5,6,7,8-tetrahydroimidazo[1,2-a]pyridin-2-carboxamid
6 (4-(2-Fluorphenyl)piperazin-1-yl)(6-(4-methoxy-3,5-dimethylphenyl)-5,6,7,8-tetrahydroimidazo[1,2-a]pyridin-2-yl)methanon
7 (6-(5-Fluor-2-methoxyphenyl)-5,6,7,8-tetrahydroimidazo[1,2-a]pyridin-2-yl)(4-(3-(trifluormethyl)phenyl)piperazin-1-yl)methanon
8 (4-Benzylpiperidin-1-yl)(6-(5-fluor-2-methoxyphenyl)-5,6,7,8-tetrahydroimidazo[1,2-a]pyridin-2-yl)methanon
9 (6-(5-Fluor-2-methoxyphenyl)-5,6,7,8-tetrahydroimidazo[1,2-a]pyridin-2-yl)(1,3,3-trimethyl-6-azabicyclo[3.2.1]octan-6-yl)methanon
10 (6-(4-Methoxy-3,5-dimethylphenyl)-5,6,7,8-tetrahydroimidazo[1,2-a]pyridin-2-yl)(4-(2-Methoxyphenyl)piperazin-1-yl)methanon
11 (4-Benzylpiperidin-1-yl)(6-(4-ethylphenyl)-5,6,7,8-tetrahydroimidazo[1,2-a]pyridin-2-yl)methanon
12 (6-(2,5-Dimethoxyphenyl)-5,6,7,8-tetrahydroimidazo[1,2-a]pyridin-2-yl)(1,3,3-trimethyl-6-azabicyclo[3.2.1]octan-6-yl)methanon
13 (6-(4-Fluor-3-methylphenyl)-5,6,7,8-tetrahydroimidazo[1,2-a]pyridin-2-yl)(4-(2-fluorphenyl)piperazin-1-yl)methanon
14 6-(4-Fluor-3-methylphenyl)-N-(1-phenylethyl)-5,6,7,8-tetrahydroimidazo[1,2-a]pyridin-2-carboxamid
15 6-(4-Methoxy-3,5-dimethylphenyl)-N-pentyl-5,6,7,8-tetrahydroimidazo[1,2-a]pyridin-2-carboxamid
16 N-Hexyl-6-(4-methoxy-3,5-dimethylphenyl)-5,6,7,8-tetrahydroimidazo[1,2-a]pyridin-2-carboxamid
17 6-(4-Methoxy-3,5-dimethylphenyl)-N-(3-(trifluormethyl)Benzyl)-5,6,7,8-tetrahydroimidazo[1,2-a]pyridin-2-carboxamid
18 N-sec-Butyl-6-(4-phenoxyphenyl)-5,6,7,8-tetrahydroimidazo[1,2-a]pyridin-2-carboxamid
19 N-Butyl-6-(4-phenoxyphenyl)-5,6,7,8-tetrahydroimidazo[1,2-a]pyridin-2-carboxamid
20 Ethyl 1-(6-(4-phenoxyphenyl)-5,6,7,8-tetrahydroimidazo[1,2-a]pyridin-2-carbonyl)piperidin-4-carboxylat

21 (4-Methylpiperidin-1-yl)(6-(4-phenoxyphenyl)-5,6,7,8-tetrahydroimidazo[1,2-a]pyridin-2-yl)methanon
22 Ethyl 1-(6-(4-phenoxyphenyl)-5,6,7,8-tetrahydroimidazo[1,2-a]pyridin-2-carbonyl)piperidin-3-carboxylat
23 1-(4-(6-(4-Phenoxyphenyl)-5,6,7,8-tetrahydroimidazo[1,2-a]pyridin-2-carbonyl)piperazin-1-yl)ethanon
24 6-(4-Phenoxyphenyl)-N-(pyridin-3-ylmethyl)-5,6,7,8-tetrahydroimidazo[1,2-a]pyridin-2-carboxamid
25 (6-(4-Phenoxyphenyl)-5,6,7,8-tetrahydroimidazo[1,2-a]pyridin-2-yl)(pyrrolidin-1-yl)methanon
26 N-Cyclopropyl-6-(4-phenoxyphenyl)-5,6,7,8-tetrahydroimidazo[1,2-a]pyridin-2-carboxamid
27 N-(3,4-Dimethoxyphenethyl)-6-(4-phenoxyphenyl)-5,6,7,8-tetrahydroimidazo[1,2-a]pyridin-2-carboxamid
28 N-Pentyl-6-(4-phenoxyphenyl)-5,6,7,8-tetrahydroimidazo[1,2-a]pyridin-2-carboxamid
29 Pyrrolidin-1-yl(6-m-tolyl-5,6,7,8-tetrahydroimidazo[1,2-a]pyridin-2-yl)methanon
30 (6-(3,5-Bis(trifluormethyl)phenyl)-5,6,7,8-tetrahydroimidazo[1,2-a]pyridin-2-yl)(pyrrolidin-1-yl)methanon
31 Pyrrolidin-1-yl(6-(3-(trifluormethyl)phenyl)-5,6,7,8-tetrahydroimidazo[1,2-a]pyridin-2-yl)methanon
32 (4-(2-Fluorphenyl)piperazin-1-yl)(6-(3-(trifluormethyl)phenyl)-5,6,7,8-tetrahydroimidazo[1,2-a]pyridin-2-yl) methanon
33 (6-(2,4-Dimethoxyphenyl)-5,6,7,8-tetrahydroimidazo[1,2-a]pyridin-2-yl)(pyrrolidin-1-yl)methanon
34 Pyrrolidin-1-yl(6-o-tolyl-5,6,7,8-tetrahydroimidazo[1,2-a]pyridin-2-yl)methanon
35 (6-o-Tolyl-5,6,7,8-tetrahydroimidazo[1,2-a]pyridin-2-yl)(4-(3-(trifluormethyl)phenyl)piperazin-1-yl)methanon
36 (6-(2,5-Difluorphenyl)-5,6,7,8-tetrahydroimidazo[1,2-a]pyridin-2-yl)(pyrrolidin-1-yl)methanon
37 (6-(4-Ethylphenyl)-5,6,7,8-tetrahydroimidazo[1,2-a]pyridin-2-yl)(pyrrolidin-1-yl)methanon
38 (6-(4-Fluorphenyl)-5,6,7,8-tetrahydroimidazo[1,2-a]pyridin-2-yl)(1,3,3-trimethyl-6-azabicyclo[3.2.1]octan-6-yl)methanon
39 6-(4-Methoxyphenyl)-N-(3-(trifluormethyl)benzyl)-5,6,7,8-tetrahydroimidazo[1,2-a]pyridin-2-carboxamid
40 6-(4-Ethylphenyl)-N-phenethyl-5,6,7,8-tetrahydroimidazo[1,2-a]pyridin-2-carboxamid
41 (4-Benzylpiperidin-1-yl)(6-(4-fluorphenyl)-5,6,7,8-tetrahydroimidazo[1,2-a]pyridin-2-yl)methanon
42 N-(Benzo[d][1,3]dioxol-5-ylmethyl)-6-(3,5-bis(trifluormethyl)phenyl)-5,6,7,8-tetrahydroimidazo[1,2-a]pyridin-2-carboxamid
43 N-Benzyl-6-(3,5-bis(trifluormethyl)phenyl)-5,6,7,8-tetrahydroimidazo[1,2-a]pyridin-2-carboxamid
44 (6-(4-Methoxy-3,5-dimethylphenyl)-5,6,7,8-tetrahydroimidazo[1,2-a]pyridin-2-yl)(4-(4-methoxyphenyl)piperazin-1-yl)methanon
45 (4-(2-Ethoxyphenyl)piperazin-1-yl)(6-m-tolyl-5,6,7,8-tetrahydroimidazo[1,2-a]pyridin-2-yl)methanon
46 (6-(2,4-Dimethoxyphenyl)-5,6,7,8-tetrahydroimidazo[1,2-a]pyridin-2-yl)(4-(3-(trifluormethyl)phenyl)piperazin-1-yl)methanon
47 (6-(4-Fluorphenyl)-5,6,7,8-tetrahydroimidazo[1,2-a]pyridin-2-yl)(4-(3-(triFluormethyl)phenyl)piperazin-1-yl) methanon
48 (6-(3-Fluorphenyl)-5,6,7,8-tetrahydroimidazo[1,2-a]pyridin-2-yl)(4-(3-(trifluormethyl)phenyl)piperazin-1-yl) methanon
49 N-(4-Fluorbenzyl)-6-(4-methoxy-3,5-dimethylphenyl)-5,6,7,8-tetrahydroimidazo[1,2-a]pyridin-2-carboxamid
50 (4-Benzylpiperidin-1-yl)(6-(3-methoxyphenyl)-5,6,7,8-tetrahydroimidazo[1,2-a]pyridin-2-yl)methanon
51 6-(3-Fluorphenyl)-N-(4-phenylbutan-2-yl)-5,6,7,8-tetrahydroimidazo[1,2-a]pyridin-2-carboxamid
52 6-(4-Ethylphenyl)-N-(4-phenylbutan-2-yl)-5,6,7,8-tetrahydroimidazo[1,2-a]pyridin-2-carboxamid
53 (6-Phenyl-5,6,7,8-tetrahydroimidazo[1,2-a]pyridin-2-yl)(4-(3-(trifluormethyl)phenyl)piperazin-1-yl)methanon
54 (6-(4-Methoxy-3,5-dimethylphenyl)-5,6,7,8-tetrahydroimidazo[1,2-a]pyridin-2-yl)(4-(3-(trifluormethyl)phenyl)piperazin-1-yl)methanon
55 (4-Benzylpiperidin-1-yl)(6-(4-methoxy-3,5-dimethylphenyl)-5,6,7,8-tetrahydroimidazo[1,2-a]pyridin-2-yl) methanon
56 (6-(4-Methoxy-3,5-dimethylphenyl)-5,6,7,8-tetrahydroimidazo[1,2-a]pyridin-2-yl)(1,3,3-trimethyl-6-azabicyclo[3.2.1]octan-6-yl)methanon
57 4-Benzylpiperidin-1-yl)(6-(4-fluor-3-methylphenyl)-5,6,7,8-tetrahydroimidazo[1,2-a]pyridin-2-yl)methanon
58 (6-(4-Fluor-3-methylphenyl)-5,6,7,8-tetrahydroimidazo[1,2-a]pyridin-2-yl)(4-(3-(triFluormethyl)phenyl)piperazin-1-yl)methanon
59 (4-Benzhydrylpiperazin-1-yl)(6-(4-methoxy-3,5-dimethylphenyl)-5,6,7,8-tetrahydroimidazo[1,2-a]pyridin-2-yl)methanon
60 6-(3-Methoxyphenyl)-N-(4-phenylbutan-2-yl)-5,6,7,8-tetrahydroimidazo[1,2-a]pyridin-2-carboxamid
61 (4-Benzylpiperidin-1-yl)(6-(3-fluorphenyl)-5,6,7,8-tetrahydroimidazo[1,2-a]pyridin-2-yl)methanon
62 (6-(4-Fluor-3-methylphenyl)-5,6,7,8-tetrahydroimidazo[1,2-a]pyridin-2-yl)(4-(4-(trifluormethyl)phenyl)piperazin-1-yl)methanon
63 (6-(4-Fluor-3-methylphenyl)-5,6,7,8-tetrahydroimidazo[1,2-a]pyridin-2-yl)(4-(4-fluorphenyl)piperazin-1-yl)methanon
64 (6-(4-Fluor-3-methylphenyl)-5,6,7,8-tetrahydroimidazo[1,2-a]pyridin-2-yl)(4-(4-methylphenyl)piperazin-1-yl)methanon
65 (6-(4-Fluor-3-methylphenyl)-5,6,7,8-tetrahydroimidazo[1,2-a]pyridin-2-yl)(4-(3-methylphenyl)piperazin-1-yl)methanon
66 (4-Butylpiperazin-1-yl)(6-(4-fluor-3-methylphenyl)-5,6,7,8-tetrahydroimidazo[1,2-a]pyridin-2-yl)methanon
67 (4-Benzhydrylpiperidin-1-yl)(6-(4-fluor-3-methylphenyl)-5,6,7,8-tetrahydroimidazo[1,2-a]pyridin-2-yl)methanon
68 N-(Cyclohexylmethyl)-6-(4-fluor-3-methylphenyl)-5,6,7,8-tetrahydroimidazo[1,2-a]pyridin-2-carboxamid
69 (6-(4-Methoxyphenyl)-5,6,7,8-tetrahydroimidazo[1,2-a]pyridin-2-yl)(4-(3-(trifluormethyl)phenyl)piperazin-1-yl)methanon
70 (4-Benzylpiperidin-1-yl)(6-m-tolyl-5,6,7,8-tetrahydroimidazo[1,2-a]pyridin-2-yl)methanon
71 (6-(3-Methoxyphenyl)-5,6,7,8-tetrahydroimidazo[1,2-a]pyridin-2-yl)(4-(3-(trifluormethyl)phenyl)piperazin-1-yl)methanon
72 6-(4-Fluor-3-methylphenyl)-N-(4-phenylbutan-2-yl)-5,6,7,8-tetrahydroimidazo[1,2-a]pyridin-2-carboxamid
73 4-Benzylpiperidin-1-yl)(6-p-tolyl-5,6,7,8-tetrahydroimidazo[1,2-a]pyridin-2-yl)methanon
74 (6-p-Tolyl-5,6,7,8-tetrahydroimidazo[1,2-a]pyridin-2-yl)(4-(3-(trifluormethyl)phenyl)piperazin-1-yl)methanon
75 (6-m-Tolyl-5,6,7,8-tetrahydroimidazo[1,2-a]pyridin-2-yl)(4-(3-(trifluormethyl)phenyl)piperazin-1-yl)methanon
76 (4-Benzylpiperidin-1-yl)(6-(4-methoxyphenyl)-5,6,7,8-tetrahydroimidazo[1,2-a]pyridin-2-yl)methanon
77 (6-(3,5-Dimethylphenyl)-5,6,7,8-tetrahydroimidazo[1,2-a]pyridin-2-yl)(4-(3-(trifluormethyl)phenyl)piperazin-1-yl)methanon
78 N-(1-(3,5-Dimethylphenyl)ethyl)-6-(4-fluor-3-methylphenyl)-5,6,7,8-tetrahydroimidazo[1,2-a]pyridin-2-carboxamid
79 6-(3,4-Difluorphenyl)-5,6,7,8-tetrahydroimidazo[1,2-a]pyridin-2-yl)(4-(3-(trifluormethyl)phenyl)piperazin-1-yl)methanon
80 (4-Benzylpiperazin-1-yl)(6-(4-fluor-3-methylphenyl)-5,6,7,8-tetrahydroimidazo[1,2-a]pyridin-2-yl)methanon
81 (6-(4-Phenoxyphenyl)-5,6,7,8-tetrahydroimidazo[1,2-a]pyridin-2-yl)(4-(3-(trifluormethyl)phenyl)piperazin-1-yl)methanon
82 6-(4-(Benzyloxy)phenyl)-5,6,7,8-tetrahydroimidazo[1,2-a]pyridin-2-yl)(4-(3-(trifluormethyl)phenyl)piperazin-1-yl)methanon

**9.** Arzneimittel enthaltend wenigstens ein substituiertes Tetrahydroimidazopyridin-Derivat gemäß Anspruch 1, sowie gegebenenfalls enthaltend geeignete Zusatz- und/oder Hilfsstoffe und/oder gegebenenfalls weiterer Wirkstoffe.

**10.** Verwendung von substituierten Tetrahydroimidazopyridin-Verbindungen gemäß Anspruch 1 zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz.

**11.** Verwendung von Verbindungen gemäß Anspruch 1 zur Herstellung eines Arzneimittels zur Behandlung von Epilepsie, Migräne, Angstzuständen und Harninkontinenz.

**12.** Verfahren zur Herstellung von Tetrahydroimidazopyridin-Derivaten der allgemeinen Formel I gemäß Anspruch 1, X = Cl, Br, I

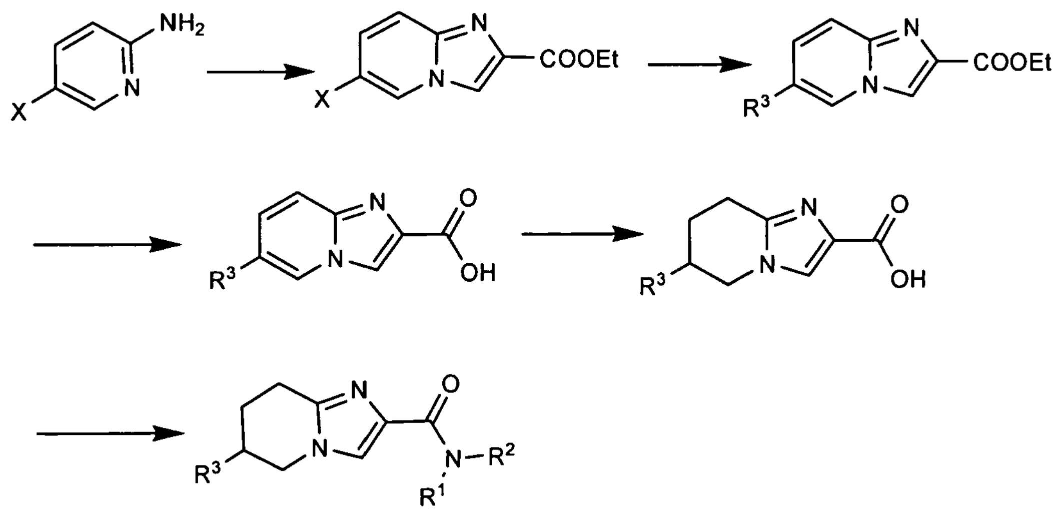

wobei

a) ein iod-, brom- oder chlorsubstituiertes 2-Aminopyridin mit 3-Bromo-2-oxopropionsäure in einem organischen Lösungsmittel, beispielsweise Ethanol, Methanol, THF, 1,2-Dimethoxyethan, Aceton, Wasser oder Chloroform bei Temperaturen zwischen 0°C und 80°C bei einer Reaktionszeit von 2-48 h umgesetzt wird,
b) der erhaltene iod-, brom- oder chlorsubstituierte Imidazo[1,2-a]pyridin-2-carbonsäureethylester mit entsprechenden Phenylboronsäuren oder Phenylboronsäureestern in Lösungsmitteln, beispielsweise Methanol, Ethanol, 1-Propanol, Ethylenglycol, Wasser, 1,2-Dimethoxyethan, THF, Dioxan, Acetonitril, DMF, Benzol, Toluol, oder Xylol, unter Verwendung eines Katalysators, einer Base und gegebenenfalls eines Additivs umgesetzt wird,
c) der Carbonsäureester unter Verwendung von organischen Säuren, beispielsweise Trifluoressigsäure, oder wässrigen anorganischen Säuren, beispielsweise Salzsäure, oder unter Verwendung von wässrigen anorganischen Basen, beispielsweise Lithiumhydroxid, Kaliumhydroxid, Natriumhydroxid, Natriumcarbonat, Natriumhydrogencarbonat, Kaliumcarbonat, in organischen Lösungsmittel, beispielsweise Methanol, Dioxan, Dichlormethan, THF oder Diethylether, gespalten wird,
d) die erhaltene substituierte Imidazopyridincarbonsäure hydriert wird, beispielsweise mit Pd auf Aktivkohle, Raney Nickel, Platin oder $PtO_2$, in einem Lösungsmittel, beispielsweise Ethanol, Methanol, Wasser, Essigsäure, Propionsäure, DCM, Cyclohexan oder methanolische KOH, gegebenenfalls unter Zusatz eines Additivs, bei Normaldruck oder erhöhtem Druck,
e) die erhaltene Tetrahydroimidazopyridincarbonsäure mit primären oder sekundären Aminen unter Verwendung von Basen, beispielsweise Natriummethanolat, TEA, DIEA oder N-Methylmorpholin, und gegebenenfalls Kupplungsreagenzien, beispielsweise EDCI, HOBt, DCC, CDI, HBTU, DMAP oder Pentafluorphenyldiphenylphosphinat, in Lösungsmitteln, beispielsweise Methanol, DMF oder DCM, umgesetzt wird.

Europäisches Patentamt

**EUROPÄISCHER RECHERCHENBERICHT**

Nummer der Anmeldung
EP 08 00 1093

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2004/024074 A (MERCK & CO INC [US]; CAMPBELL BRIAN THOMAS [US]; MUNOZ BENITO [US]; ST) 25. März 2004 (2004-03-25)<br>* Ansprüche 15,16,22; Beispiel 5 *<br>----- | | INV.<br>C07D471/04<br>A61K31/437<br>A61P25/00 |
| A | DE 10 2004 037445 A1 (SANOL ARZNEI SCHWARZ GMBH [DE]) 16. März 2006 (2006-03-16)<br>* Seite 14, Absätze 93,94,96; Anspruch 1 *<br>----- | | |
| A | US 2005/154202 A1 (HAGMANN WILLIAM K [US] ET AL) 14. Juli 2005 (2005-07-14)<br>* das ganze Dokument *<br>----- | | |
| A | GB 1 473 819 A (GALLARDO ANTONIO SA) 18. Mai 1977 (1977-05-18)<br>* das ganze Dokument *<br>----- | | |
| | | | RECHERCHIERTE SACHGEBIETE (IPC) |
| | | | C07D<br>A61K |

Der vorliegende Recherchenbericht wurde für alle Patentansprüche erstellt

| Recherchenort | Abschlußdatum der Recherche | Prüfer |
|---|---|---|
| Den Haag | 31. Juli 2008 | Skulj, Primoz |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................................................................
& : Mitglied der gleichen Patentfamilie, übereinstimmendes Dokument

4

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 08 00 1093

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-07-2008

| Im Recherchenbericht angeführtes Patentdokument | Datum der Veröffentlichung | Mitglied(er) der Patentfamilie | Datum der Veröffentlichung |
|---|---|---|---|
| WO 2004024074 A | 25-03-2004 | AU 2003267087 A1 | 30-04-2004 |
| | | CA 2498237 A1 | 25-03-2004 |
| | | EP 1539749 A2 | 15-06-2005 |
| | | JP 2006503038 T | 26-01-2006 |
| DE 102004037445 A1 | 16-03-2006 | CN 101014599 A | 08-08-2007 |
| US 2005154202 A1 | 14-07-2005 | KEINE | |
| GB 1473819 A | 18-05-1977 | DE 2702790 A1 | 04-08-1977 |
| | | ES 454823 A1 | 01-01-1978 |
| | | ES 454824 A1 | 01-01-1978 |
| | | FR 2339612 A1 | 26-08-1977 |
| | | JP 52116494 A | 29-09-1977 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20020128277 A
- WO 2006015737 A


### In der Beschreibung aufgeführte Nicht-Patentliteratur


- **PASSMORE et al.** *J Neurosci.,* 2003, vol. 23 (18), 7227-36
- **BLACKBURN-MUNRO ; JENSEN.** *Eur J Pharmacol.,* 2003, vol. 460 (2-3), 109-16
- **DOST et al.** *Naunyn Schmiedebergs Arch Pharmacol,* 2004, vol. 369 (4), 382-390
- **NIELSEN et al.** *Eur J Pharmacol.,* 2004, vol. 487 (1-3), 93-103
- **GRIBKOFF.** *Expert Opin Ther Targets,* 2003, vol. 7 (6), 737-748
- **KORSGAARD et al.** *J Pharmacol Exp Ther.,* 2005, vol. 14 (1), 282-92
- **WICKENDEN et al.** *Expert Opin Ther Pat,* 2004, vol. 14 (4), 457-469
- **STRENG et al.** *J Urol,* 2004, vol. 172, 2054-2058
- **DE SARRO et al.** *Naunyn-Schmiedeberg's Arch. Pharmacol.,* 2001, vol. 363, 330-336